# EUROPEAN PATENT APPLICATION

(11) **EP 4 631 515 A1**
(43) Date of publication of application: **15.10.2025**
(21) Application number: 24382382.0
(22) Date of filing: 12.04.2024
(51) Int. Cl.: A61K 38/00, A61K 38/16, C12N 15/52, C12N 15/86

(54) **ANTITUMOR PARASPORIN-2 DERIVED TOXINS**

(71) Applicant: Fundació Institut d'Investigació Biomèdica de Bellvitge (IDIBELL), 08908 L'Hospitalet de Llobregat (Barcelona) (ES); Institut Català d'Oncologia (ICO), 08908 L'Hospitalet de Llobregat (ES)
(72) Inventor: ALEMANY BONASTRE, Ramon, E-08860 Castelldefels, Barcelona (ES)
(74) Representative: ABG Intellectual Property Law, S.L.

(57) **Abstract**

The invention relates to a recombinant parasporin 2 protein comprising one heterologous protease cleavage site to allow the removal of the N-terminal sequence that inhibits the pore-forming ability and, optionally, a further heterologous protease cleavage site to allow the removal of the C-terminal sequence that inhibits the pore-forming ability, and to the medical uses thereof.

## Description

### TECHNICAL FIELD

The present invention relates to the field of treating cancer.

### BACKGROUND ART

Different types of toxins have been used to kill tumor cells. Some of these proteins, such as *Pseudomonas aeruginosa* Exotoxin A, ricin, and diphtheria toxin, elicit their function intracellularly, for example by degrading RNA or blocking ribosomes. These intracellular toxins must enter the tumor cell to reach their targets. Other toxins, such as pore-forming toxins (PFTs), can elicit their toxic function without the need to enter the cell, acting at the plasma membrane level. PFTs are synthesized as soluble protoxins and activated by a proteolytic cleavage that removes one or several inhibitory domains located at the N-terminus or C-terminus of the protoxin. Upon this cleavage-mediated activation, the mature toxin binds a receptor in the cell membrane, oligomerizes and crosses the cell membrane to form a pore that perforates the membrane. Insertion into the membrane requires a large conformational change from the protoxin to the toxin structures in which a domain of the protein extends and penetrates the membrane.

The list of PFTs of bacterial origin is extensive. According to the structure of the transmembrane region they can be grouped into two classes: alpha class when it is an alpha-helix or beta class when it is an amphipathic beta-strand. Each class is further divided in numerous families, each one characterized by a similar structure of the toxin and the pore formed upon oligomerization. Among the most studied beta-PFTs are alpha-hemolysin from *Staphylococcus aureus,* alpha-toxin from *Clostridium septicum,* enterotoxin from *Clostridium perfringens,* and aerolysin from *Aeromonas hydrophila.*

The use of protease-sensitive linkers to cleave and activate cytotoxic drugs, peptides or proteins in the tumor microenvironment has been described since 1980s.

A small toxic peptide from bee venom named mellitin has been modified to be active upon cleavage by fibroblast-activation protein (FAP). The protective antigen of anthrax toxin has been modified with matrix metalloproteinase (MMP) or urokinase-type plasminogen activator (uPA) cleavage sites for activation (see Dudani et al Annual Review of Cancer Biology Vol. 2:353-376 (March 2018)). Aerolysin is naturally processed by several proteases (mainly furin) to release a C-terminal inhibitory domain. Another beta-PFT that has been modified with a MMP-2 proteolytic site to be activated in the tumor microenvironment is alpha-hemolysin from *Staphylococcus aureus* (Koo et al ACS Cent. Sci. 2019, 5, 4, 629-639). In this case the toxin was split into two halves and a protease-recognition site was introduced that extends stem domains that need to be removed for these two halves to assemble.

*Bacillus thuringiensis* is a Gram-positive bacterium that upon sporulation produces a crystalline parasporal inclusion or paraspore. The paraspore contains proteins of two major classes, Cry (Crystal) and Cyt (cytotoxic to Dipteran larvae). Most of these proteins have hemolytic and insecticidal activities. However, some are non-hemolytic, can kill cancer cells and are known as parasporins (Mizuki et al Clin. Diagn. Lab. Immunol. 2000, 7 (4), 625-634; Santos et al. Biochimie 2022 Biochimie 2022, 192, 83-90). Despite the reported lytic effect of parasporins in tumor cells *in vitro,* the evidence of antitumor activity *in vivo* is missing.

Previous reports have used the proteinase-K activated parasporin 2 to kill tumor cells *in vitro.* To perform these cell-killing assays parasporin 2 is purified from bacteria, cleaved with proteinase K, and the resulting cleaved and activated toxin is purified again. However, proteinase K cannot be used to activate this toxin in the tumor microenvironment since this broad-spectrum protease would degrade all proteins in the tissue.

Therefore, there is a need in the art to provide alternative parasporins which can be used for treating cancer.

### SUMMARY OF THE INVENTION

The present invention in embodiments seeks to obtain an activated parasporin 2 without the need to treat parasporin 2 with proteinase K. This invention inter alia aims to produce the parasporin 2 protoxin *in situ* in the tumor and allow for intratumoral activation by proteases present in the tumor.

In a first aspect, the invention relates to a recombinant parasporin 2 protein comprising one heterologous protease cleavage site to allow the removal of the N-terminal sequence that inhibits the pore-forming ability and, optionally, a further heterologous protease cleavage site to allow the removal of the C-terminal sequence that inhibits the pore-forming ability.

In another aspect, the invention relates to a nucleic acid encoding a recombinant parasporin 2 protein according to the invention.

In another aspect, the invention relates to an expression cassette comprising the nucleic acid according to the invention.

In another aspect, the invention relates to a vector comprising the expression cassette according to the invention.

In another aspect, the invention relates to a cell comprising a vector according to the invention.

In another aspect, the invention relates to a pharmaceutical composition comprising a recombinant parasporin 2 protein according to the invention, a nucleic acid according to the invention, an expression cassette according to the invention, a vector according to the invention or a cell according to the invention and a pharmaceutically acceptable carrier or excipient.

In another aspect, the invention relates to a recombinant parasporin 2 according to the invention, a nucleic acid according to the invention, an expression cassette according to the invention, a vector according to the invention, a cell according to the invention or a pharmaceutical composition according to the invention for use in medicine.

In another aspect, the invention relates to a recombinant parasporin 2 according to the invention, a nucleic acid according to the invention, an expression cassette according to the invention, a vector according to the invention, a cell according to the invention or a pharmaceutical composition according to the invention for use in the prevention and/or treatment of cancer.

### DESCRIPTION OF THE FIGURES

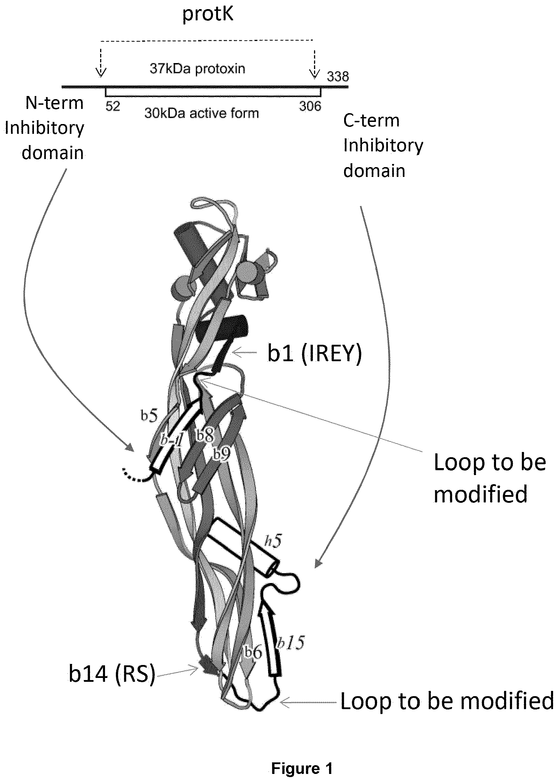

**Figure 1****.** Top: Linear representation of the parasporin 2 (PS2) wild type sequence showing the inactive protoxin of 37KDa and the active protein of 30KDa that is generated upon cleavage of the protoxin at the two flanking indicated sites by proteinase K (protK). Bottom: Structure of PS2 monomer before activation (protoxin) with the two inhibitory terminal regions in white. The loops preceding the first beta sheet (b1) and after the last beta sheet (b14) that are modified in the present invention are indicated.

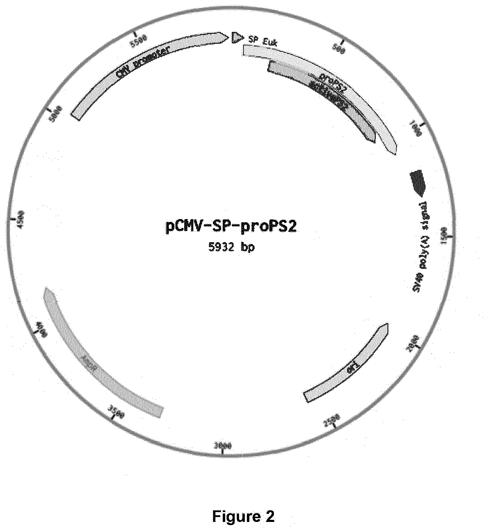

**Figure 2****.** Plasmid representation of a cloned parasporin 2 (PS2) to be expressed in eukaryotic cells. The wild type PS2 sequence has been modified with the insertion of a signal peptide (SP) for secretion in eukaryotic cells. The CMV promoter and the SV40 pA signals are used to generate the mRNA.

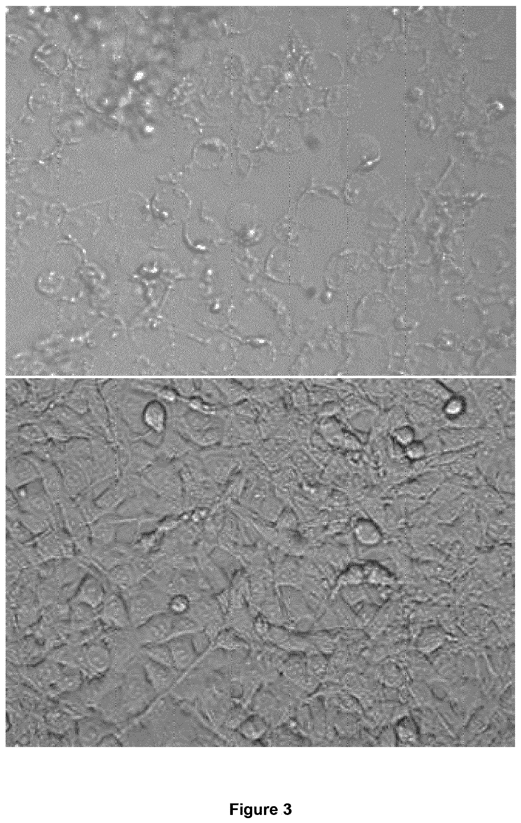

**Figure 3****.** Cytotoxic effect of a wild type PS2Aa1 secreted from a eukaryotic cell. HEK293 cells were transfected with an expression plasmid encoding wild type PS2Aa1 with a signal peptide. The supernatant from transfected cells was harvested 48h post-transfection. This supernatant was incubated during 1h at 37°C with proteinase K (protK) at a final concentration of 100 µg/ml to activate the protoxin into a toxin. ProtK was inactivated by adding 1mM of PMSF. The protK-treated supernatant was added to CT26 cells and pictures of cells were taken 24h later. Top: CT26 colon cancer cells incubated with the protK-treated supernatant from HEK293 cells transfected with the expression plasmid of PS2Aa1. Cellular cytotoxicity induced by the toxin is observed as a cell swelling or ballooning morphological change in the cells as previously described: cells become rounded and swollen. Cell membranes show blebs or blebbing. Bottom: untreated CT26 cells.

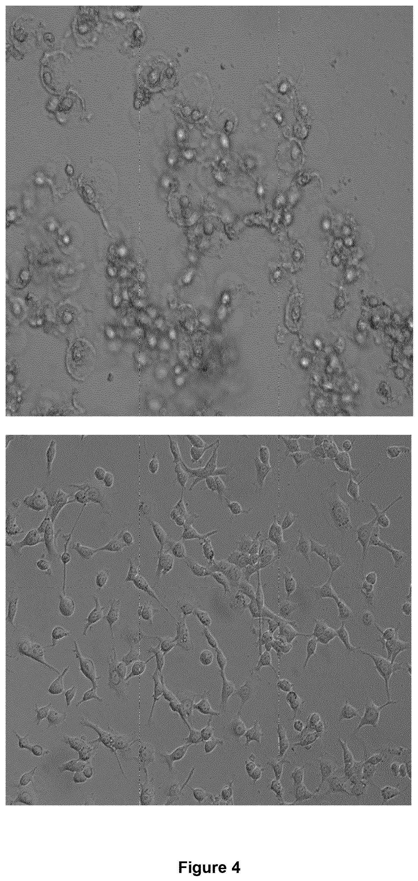

**Figure 4****.** Cytotoxic effect of HPS2Aa1 secreted from a eukaryotic cell. HEK293 cells were transfected with an expression plasmid encoding HPS2Aa1 with a signal peptide. The supernatant from transfected cells was harvested 48h post-transfection. This supernatant was incubated during 1h at 37°C with Human Rhinovirus 3C (HRV3C) protease at a final concentration of 10 units/ml to activate the protoxin into a toxin. The HRV3C-treated supernatant was added to CT26 cells and a picture of cells was taken 24h later. Top: CT26 colon cancer cells incubated with the supernatant from HEK293 cells transfected with the expression plasmid of HPS2Aa1. Cytotoxicity is observed with the characteristic morphological changes associated to parasporin-2 cytotoxicity as described for figure 3. Bottom: untreated CT26 cells.

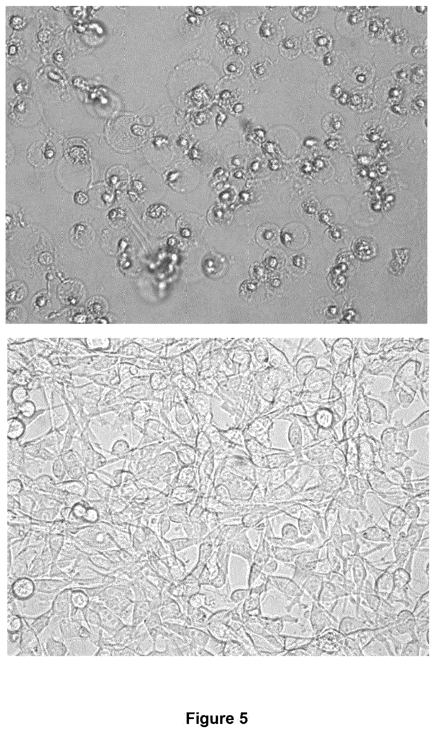

**Figure 5****.** Cytotoxic effect of a PS2Aa1-3 secreted from a eukaryotic cell on CT26 cells. HEK293 cells were transfected with an expression plasmid encoding PS2Aa1-3 with a signal peptide. The supernatant from transfected cells was harvested 48h post-transfection. This supernatant was added to CT26 cells and a picture of cells was taken 24h later. Top: CT26 colon cancer cells incubated with the supernatant from HEK293 cells transfected with the expression plasmid of PS2Aa1-3. Cytotoxicity is observed with the characteristic morphological changes associated to parasporin-2 cytotoxicity as described for figure 3. Bottom: untreated CT26 cells.

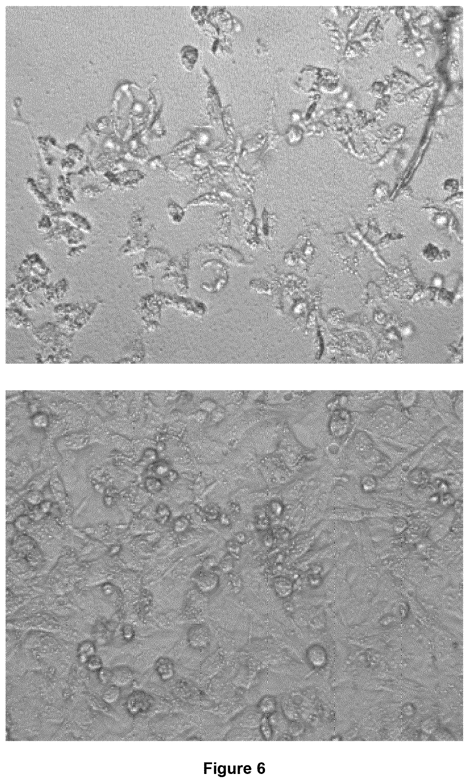

**Figure 6****.** Cytotoxic effect of a PS2Aa1-3 secreted from a eukaryotic cell on HepG2 cells. HEK293 cells were transfected with an expression plasmid encoding PS2Aa1-3 with a signal peptide. The supernatant from transfected cells was harvested 48h post-transfection. This supernatant was added to HepG2 cells and a picture of cells was taken 24h later. Top: HepG2 hepatocellular cancer cells incubated with the supernatant from HEK293 cells transfected with the expression plasmid of PS2Aa1-3. Cytotoxicity is observed with the characteristic morphological changes associated to parasporin-2 cytotoxicity as described for figure 3. Bottom: untreated HepG2 cells.

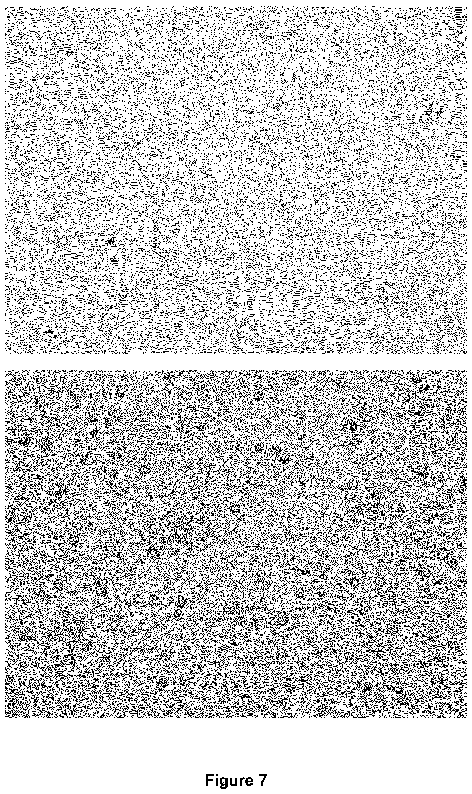

**Figure 7****.** Cytotoxic effect of a PS2Aa1-3 treated with recombinant matriptase on HeLa target cells. HEK293 cells were transfected with an expression plasmid encoding wild type PS2Aa1-3 with a signal peptide. The supernatant from transfected cells was harvested 48h post-transfection. This supernatant was incubated during 1h at 37°C with matriptase at a final concentration of 10 units /ml to activate the protoxin into a toxin. The matriptase-treated supernatant was added to HeLa cells and pictures of cells were taken 24h later. Top: HeLa cervical carcinoma cells incubated with the matriptase-treated supernatant from HEK293 cells transfected with the expression plasmid of PS2Aa1-3. Cytotoxicity is observed with the characteristic morphological changes associated to parasporin-2 cytotoxicity as described for figure 3. Bottom: untreated HeLa cells.

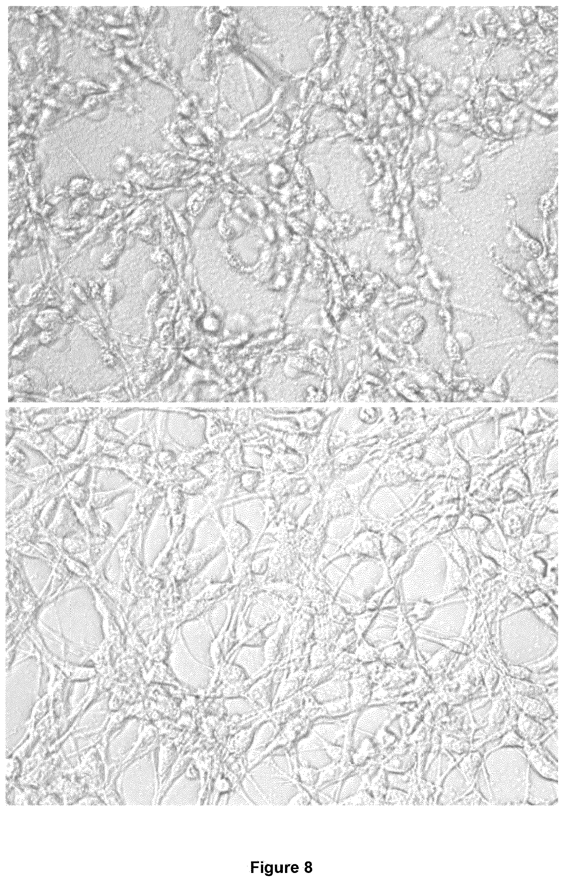

**Figure 8****.** Cytotoxic effect of PS2Aa1-1 secreted from a eukaryotic cell. HEK293 cells were transfected with an expression plasmid encoding wild type PS2Aa1-1 with a signal peptide. The supernatant from transfected cells was harvested 48h post-transfection. This supernatant was incubated during 1h at 37°C with trypsin at a final concentration of 10 µg/ml to activate the protoxin into a toxin. Trypsin was inactivated by adding fetal bovine serum (FBS) to a final concentration of 10%. The trypsin-treated supernatant was added to CT26 cells and pictures of cells were taken 24h later. Top: CT26 colon cancer cells incubated with the trypsin-treated supernatant from HEK293 cells transfected with the expression plasmid of PS2Aa1-1. Cytotoxicity is observed with the characteristic morphological changes associated to parasporin-2 cytotoxicity as described for figure 3. Bottom: untreated CT26 cells.

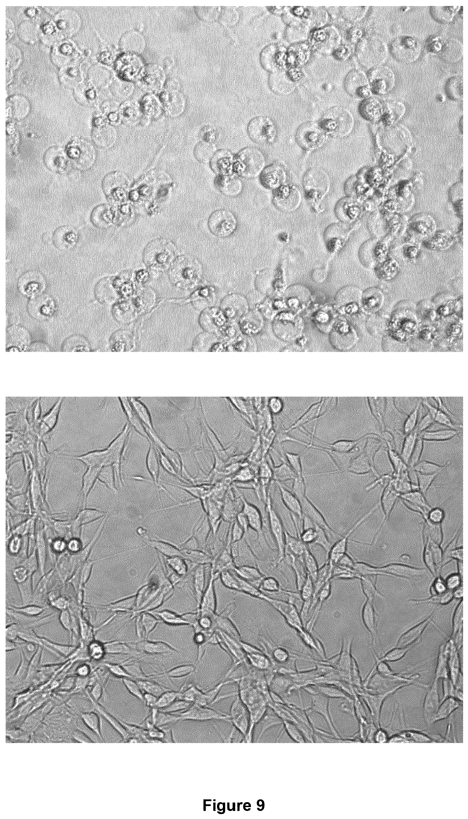

**Figure 9****.** Cytotoxic effect of PS2Aa1-Fu and PS2Aa1-Fu2 secreted from a eukaryotic cell. HEK293 cells were transfected with an expression plasmid encoding wild type PS2Aa1-Fu or PS2Aa1-Fu2 with a signal peptide. The supernatant from transfected cells was harvested 48h post-transfection. The supernatant was added to CT26 cells and pictures of cells were taken 24h later. Top: CT26 colon cancer cells incubated with the supernatant from HEK293 cells transfected with the expression plasmid of PS2Aa1-Fu. Cytotoxicity is observed with the characteristic morphological changes associated to parasporin-2 cytotoxicity as described for figure 3. Bottom: untreated CT26 cells.

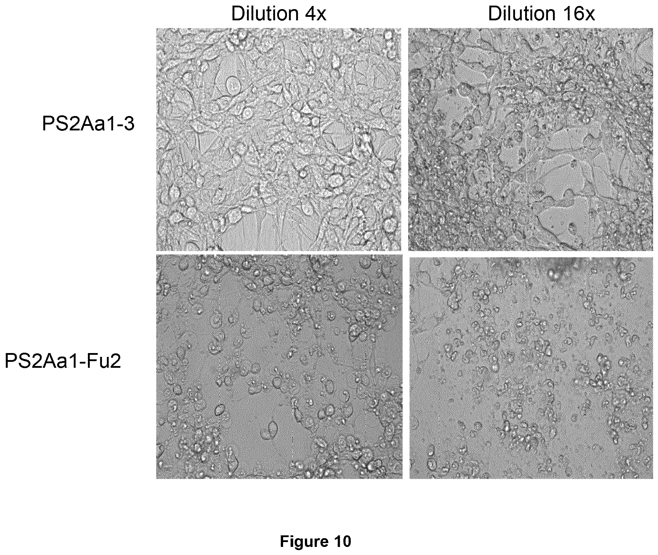

**Figure 10****.** Cytotoxic effect of PS2Aa1-3 of PS2Aa1-Fu2-containig supernatants used at different dilutions. HEK293 cells were transfected with an expression plasmid encoding wild type PS2Aa1-3 or PS2Aa1-Fu2 with signal peptides. The supernatants from transfected cells were harvested 48h post-transfection. The supernatants were diluted in DMEM 10% FBS and added to CT26 cells and pictures of cells were taken 24h later. Top: CT26 colon cancer cells incubated with the supernatant from HEK293 cells transfected with the expression plasmid of PS2Aa1-3 at 4x and 16x dilutions. No clear cytotoxicity was observed. Bottom: CT26 colon cancer cells incubated with the supernatant from HEK293 cells transfected with the expression plasmid of PS2Aa1-Fu2 at 4x and 16x dilutions. Cytotoxicity is observed with the characteristic morphological changes associated to parasporin-2 cytotoxicity as described for figure 3.

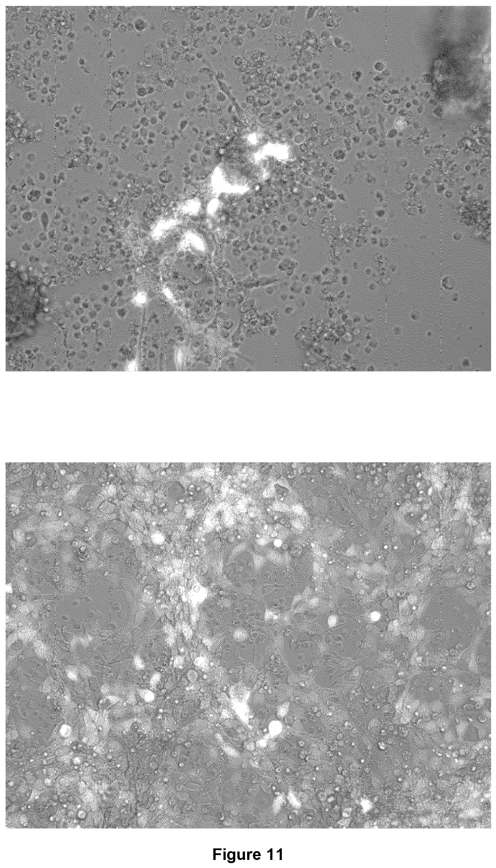

**Figure 11****.** Cytotoxic effect of PS2Aa1-Fu2-secreting HEK293-GL cells mixed with bystander CT26 tumor cells. pCMV-PS2Aa1-Fu2 plasmid was transfected into HEK293-GL human kidney cells using the calcium phosphate HBS method. As a control, pCMV-PS2Aa1 plasmid encoding the secreted wild type PS2Aa1 that needs proteinase K to be activated was used. Transfected HEK293-GL cells were co-cultured with CT26 cells for two days. Pictures were taken under fluorescence and bright field. Top: complete lysis of CT26 bystander target cells up on the coculture of PS2Aa1-Fu2-transfected HEK293-GL cells. Bottom: viable coculture of PS2Aa1-transfected HEK293-GL cells with bystander CT26 target cells.

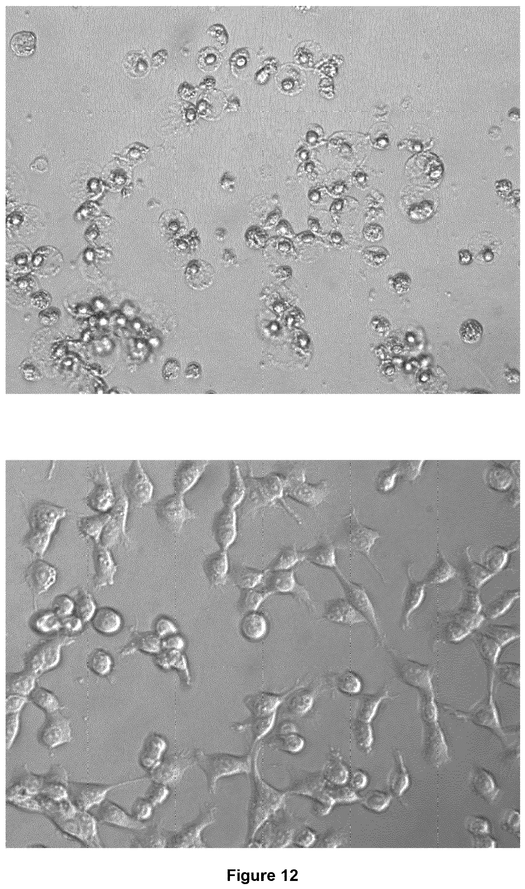

**Figure 12****.** Cytotoxic effect of PS2Ab-Fu2 secreted from a eukaryotic cell. HEK293 cells were transfected with an expression plasmid encoding PS2Ab-Fu2 with a signal peptide. The supernatant from transfected cells was harvested 48h post-transfection. The supernatant was added to CT26 cells and pictures of cells were taken 24h later. Top: CT26 colon cancer cells incubated with the supernatant from HEK293 cells transfected with the expression plasmid of PS2Ab-Fu2. Cytotoxicity is observed as cell ballooning and lysis Bottom. CT26 incubated with supernatant from untransfected HEK293 cells.

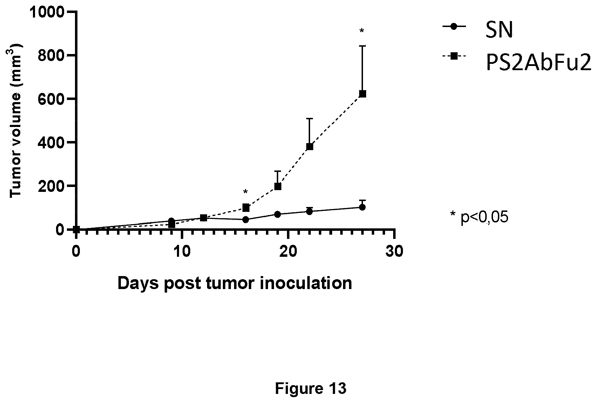

**Figure 13****.** *In vivo* antitumor effect of PS2Ab-Fu2 secreted from a eukaryotic cell. CMT64 mouse lung cancer cells were injected in C57BL/6 immune-competent mice subcutaneously and allowed to form tumors. When tumors reached a volume of 0.1 cc were injected with 20 microliters of the supernatant containing the toxin or the supernatant control. These supernatants were produced as follows: HEK293 cells were transfected with an expression plasmid encoding PS2Ab-Fu2 with a signal peptide using the calcium phosphate HBS method. One day after transfection the cell culture medium DMEM with 10% FBS was replaced with new medium. One day later this complete medium was replaced with serum-free medium (Optimem, Gibco). Two days later this supernatant containing the secreted PS2Ab-Fu2 toxin was harvested. In parallel a supernatant control was produced from HEK293 cells transfected with a control plasmid. Each treatment group, PS2Aa1 Fu2 or control, contained 5 mice with tumors. Tumor volume was followed during time. Tumors injected with the supernatant containing the modified parasporin-2 toxin PS2Ab-Fu2 were inhibited in their growth compared to the ones treated with the control.

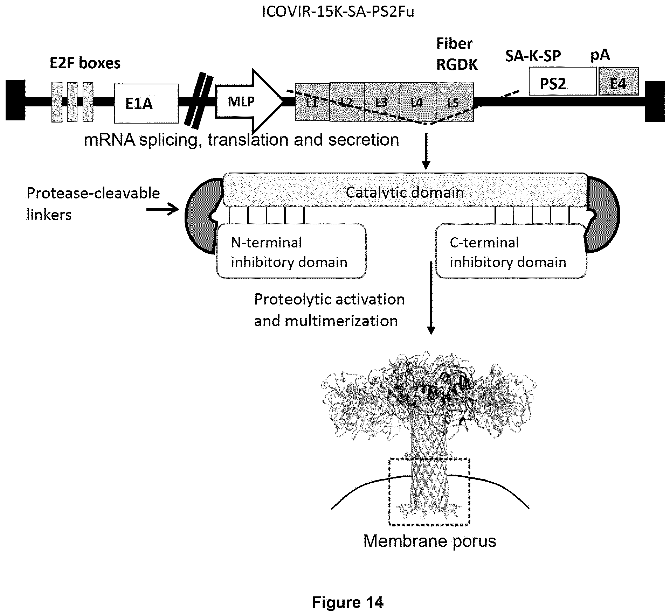

**Figure 14****.** Schematic representation of the oncolytic adenovirus ICOVIR-15K-SA-PS2Fu and the mechanism of action from the toxin secreted by a cell infected with this virus. The PS2Ab-Fu2 expression cassette is composed of an splicing acceptor (SA) and the DNA sequence encoding a modified parasporin-2. This cassette is inserted after the fiber gene of the oncolytic adenovirus ICOVIR15K using standard techniques, Rojas J. et al., Gene Ther . 2012 Apr;19(4):453-7 . This virus produces a secreted modified PS2-Ab toxin that is activated upon cleavage with furin. This activation leads to the oligomerization of the protein to form pores in the membrane of eukaryotic cells.

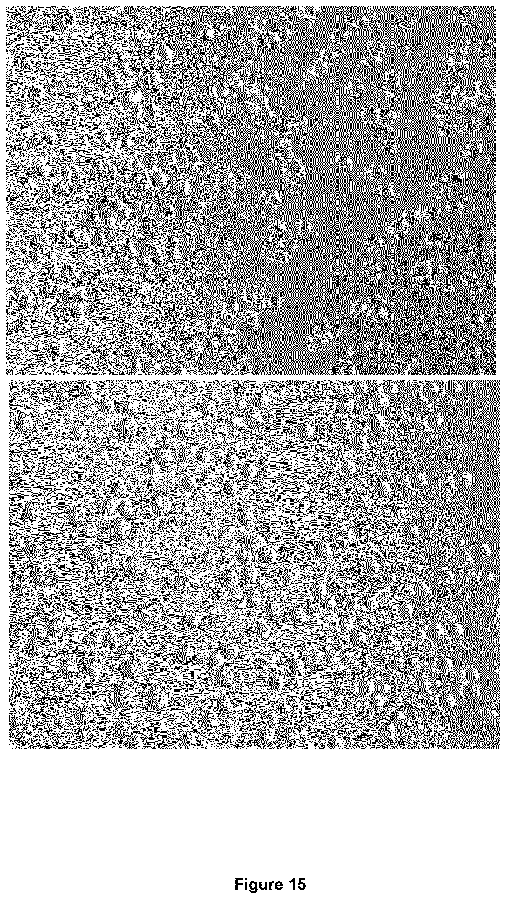

**Figure 15****.** Antitumor activity of the toxin derived from eukaryotic cells infected with the oncolytic virus ICOVIR-15K-SA-PS2Fu. A549 human lung tumor cells were infected with ICOVIR-15K-PS2Fu and three days later the supernatant was collected. A parallel infection was performed with ICOVIR15K as a control. The harvested supernatants were added to human T cell leukemia Jurkat cells. Twenty hours later pictures were taken. Supernatants derived from cells infected with ICOVIR-15K-SA-PS2Fu induced cell blebbing and cytoplasm release in Jurkat cells compared to the SN of ICOVIR15K.

### DESCRIPTION OF THE INVENTION

The author of the present invention has developed, inter alia, a recombinant parasporin 2 modified to contain one or two novel cleavage sites for proteases present in the tumor.

### Compounds of the invention

In a first aspect, the invention relates to a recombinant parasporin 2 protein comprising one heterologous protease cleavage site to allow the removal of the N-terminal sequence that inhibits the pore-forming ability and, optionally, a further heterologous protease cleavage site to allow the removal of the C-terminal sequence that inhibits the pore-forming ability.

"Recombinant protein", as used herein, relates to a protein resulting from the expression of a recombinant DNA consisting of DNA molecules formed by laboratory methods of genetic recombination (such as molecular cloning) to bring together genetic material from multiple sources, creating sequences that would not otherwise be found in the genome.

"Parasporin 2" or PS2, as used herein, relates to a protein that exhibits cytocidal activity by pore-forming mechanism only when degraded by proteases into smaller molecules of 40 to 60 kDa. Trypsin and proteinase K activate parasporin, while chymotrypsin does not.

Depending on the B. *thuringiensis* strain, the sequences of parasporin 2 (PS2) have some variation, although preserving more than 80% of sequence homology. These parasporin 2 variants include 3 different parasporins: PS2Aa1 (also known as Cry46Aa1, from strain A1547. Ito et al J. Biol. Chem. 279 (20) 21282-21286, 2004. GenBank GenPep Ref BAC79010.1, 5 October 2006). A second one named PS2Aa2 (also known as Cry46As2), from strain A1470 with reference in GenBank BAG68906.1, 24 October 2013 described in Okumura et al Biotechnol. Lett. 2013, 35 (11), 1889-1894). A third PS2 has been described, PS2Ab1 (also known as PS2Ab, Cry46Ab, from strain TK-E6. GenBank GenPep ref BAD35170.1, 26 July 2016 described by Hayakawa et al Curr. Microbiol. 2007, 55 (4), 278-283.). Both PS2Aa1 and PS2Aa2 have 338 amino acids and they differ only in 4 amino acids. PS2Ab shows a very high homology to PS2Aa1 and PS2Aa2 with the exception of having shorter inhibitory domains. Below it is shown a sequence alignment of PS2Aa1 (SEQ ID NO: 1), PS2Aa2 (SEQ ID NO: 20) and PS2Ab (SEQ ID NO: 14)
Upper lane PS2Ab (UniProt Ref Q6AW28) SEQ ID NO: 14 Middle lane PS2Aa2(UniProt Ref B5BUV6)SEQ ID NO:20 Lower lane PS2Aa1(UniProt Ref Q7WZI1)SEQ ID NO: 1 Asterisk below means complete sequence homology. Dots indicate partial homology.

                        MYYTTQVTGGFQADLNNQVVETFQPS
MYNDKRMCSDPYQGMNKPHYCNCHTYNNSEITGGLQVDLDNQVAETFQST
MYNDKRMCSDPYQGMNKPHYCNCHTYNNSEITGGLQVDLDNQVVETFQST
                        *  .:::***:*.**:***.****  :

TNVIQEYLTFNDLPALGSSPQSVRSRFSSIYGTNPDGIALNNETYFSAVQ
TDVIREYLMFNELSALSSSPESVRSRFFSIYGTNPDGIALNNETYFNAVK
TDVIREYLMFNELSALSSSPESVRSRFSSIYGTNPDGIALNNETYFNAVK
*:**:*** **:* **.***:****** ******************.**:

PPITVQYGHYCYKNVGTVQYVNRPTDINPNVILAQDTLTNNTNEPFTTTI
PPITAQYGYYCYKNVGTVQYVNRPTDINPNVILAQDTLTNNTNEPFTTTI
PPITAQYGYYCYKNVGTVQYVNRPTDINPNVILAQDTLTNNTNEPFTTTI
****.***:*****************************************

TLTGSWTKSSTVTSSTTTGLKITTKLSIKKVFEIGGEVSFSTTIGSSEAT
TITGSFTNTSTVTSSTTTGFKFTSKLSIKKVFEIGGEVSFSTTIGTSETT
TITGSFTNTSTVTSSTTTGFKFTSKLSIKKVFEIGGEVSFSTTIGTSETT
*:***:*::**********:*:*:*********************:**:*

SETFTVSKAVTVTVPAQSRRNIQLTAKIARESADFSAPITVDGYFGANFP
TETITVSKSVTVTVPAQSRRTIQLTAKIAKESADFSAPITVDGYFGANFP
TETITVSKSVTVTVPAQSRRTIQLTAKIAKESADFSAPITVDGYFGANFP
:**:****:***********.********:********** *********

RRVGPGGHYFWFNPARDVLNATSGTLRGTVTNVSSFDFQTVVQPAYSLLA
KRVGPGGHYYWFNPARDVLNTTSGTLRGTVTNVSSFDFQTIVQPARSLLD
KRVGPGGHYFWFNPARDVLNTTSGTLRGTVTNVSSFDFQTIVQPARSLLD
:********:**********:*******************:**** ***:

EQQEALESAISGDPSEEQLKQIQQTIGL----------304
EQQETLEYAIPGDPSGEQLQQMEQRMFFSQCQCPKWGN338
EQQETLEYAIPGDPSGEQLQQMEQRMFFSKCQCPKWGN338
****:** ** **** ***:*::* : :

### Percent Identity Matrix

| | PS2Ab | PS2Aa1 | PS2Aa2 |
|---|---|---|---|
| PS2Ab | **100.00%** | **82.89%** | **81.91%** |
| PS2Aa1 | **82.89%** | **100.00%** | **98.82%** |
| PS2Aa2 | **81.91%** | **98.82%** | **100.00%** |

In a preferred embodiment, parasporin is PS2Aa1 and relates to the protein from B. *thuringiensis* of strain A1547 serovar *dakota* and having a size of 37kDa (338 aa) and is cleaved at its two ends (N and C terminal) by proteinase K, to generate the active form of 30kDa. The sequence of PS2Aa1 is shown in SEQ ID NO: 1 and corresponds to the sequence shown in the GenBank database number AB099515.1 dated 5, October 2006 and GenPep database of GenBank BAC79010.1, 5 October 2006. Wild type parasporin 2 PS2Aa1 has inhibitory domains at both ends of the protein and is cleaved by proteinase K.

The term "N-terminally" or "N-terminus" or "amino-terminus" or "NH₂-terminus" or "N-terminal end" or "amine-terminus" is understood as the start of an amino acid sequence of a protein or polypeptide referring to the free amine group (-NH₂) located at one end of a polypeptide. Therefore, the N-terminal sequence relates to a sequence located near the N-terminus of the sequence. The N-terminal cleaved fragment or inhibitory domain contains the first 51 amino acid residues of parasporin. In a preferred embodiment, the N-terminal inhibitory sequence of PS2Aa1 is the sequence shown in SEQ ID NO: 18.
MYNDKRMCSDPYQGMNKPHYCNCHTYNNSEITGGLQVDLDNQVVETFQSTT (SEQ ID NO: 18)

The N-terminal sequence of the mature PS2Aa1 active protein has been determined as DVIRE (SEQ ID NO: 33). Cleavage of the N-terminal segment is essential for toxicity and the additional removal of the C-terminal segment accelerates this cytotoxicity.

The term "C-terminus" or "carboxyl-terminus" or "carboxy-terminus" or "C-terminal tail" or "C-terminal end" or "COOH-terminus" is the end of an amino acid chain of a protein or polypeptide terminated by a free carboxyl group (-COOH). Therefore the C-terminal sequence refers to a sequence located near the C-terminus of the sequence. In a preferred embodiment, the C-terminal inhibitory sequence of PS2Aa1 is the sequence shown in SEQ ID NO: 19.
QETLEYAIPGDPSGEQLQQMEQRMFFSKCQCPKWGN (SEQ ID NO: 19)

The cleavage at the C-terminus is less well defined. From the molecular weight of the mature PS2Aa1 toxin it has been calculated that 36 or more residues are released. Therefore the cleavage occurs in a position around Q302 (the length of the protoxin is 338 aa) or around Q302 to L306 (Kitada et al. J. Biol. Chem. 2006, 281 (36), 26350-26360). The active form is very toxic to certain tumor lines and has less toxicity in normal cells such as hepatocytes, so it has been proposed for use as an antitumor toxin (Saberon et al. Insect Biochem and Mol Biol 2018, 93, 66-78).

Therefore the mature active toxin comprises amino acids from D52 to Q302/L306 of SEQ ID NO: 1. Figure 1 represents this cleavage and at the bottom shows the sequences cleaved out, shown underlined, at both termini. In sequence SEQ ID NO: 14, corresponding to PS2Ab, the mature active protein comprises amino acids from D28 to Q282/L286.

"Pore-forming ability", relates to the ability of forming pores that disrupt the membrane permeability barrier and ion homeostasis and generally induce cell death. The ability of a parasporin to form a pore can be assayed using any method known in the art. For instance, the parasporin variant may be inserted into an amphiphilic layer, such as a triblock copolymer membrane, along with other appropriate subunits, and its ability to oligomerise to form a pore may be determined. Methods are known in the art for inserting subunits into amphiphilic layers, such as triblock copolymer membranes. Another common method to study pore formation is electron microscopy of lipid bilayers incubated with the toxin. Another method to measure pore formation is to measure the efflux of intracellular molecules, for example lactate dehydrogenase (LDH) or immunostimulatory histone binding protein (HMGB1), upon the incubation of cells with the toxin.

In a preferred embodiment, the recombinant parasporin 2 protein of the invention further comprises a signal peptide in the same open reading frame as the polypeptide. In a preferred embodiment, the signal peptide is a secretion signal peptide.

The term "signal peptide" refers to a short peptide, commonly 5-30 amino acids long, present at the N-terminus of newly synthesized proteins that are directed towards the secretory pathway. These proteins include those that reside either inside certain organelles (for example, the endoplasmic reticulum, Golgi or endosomes), are secreted from the cell, and transmembrane proteins. Signal peptides commonly contain a core sequence which is a long stretch of hydrophobic amino acids that tends to form a single alpha-helix. The signal peptide may begin with a short positively charged stretch of amino acids, which helps to enforce proper topology of the polypeptide during translocation. At the end of the signal peptide there is typically a stretch of amino acids that is recognized and cleaved by signal peptidase. A very large number of signal peptides for insects, yeasts, plant and mammalian cells are known, and are available in databases. For example, Signal peptide website confirmed mammalian signal peptides in its database. The invention contemplates the use of any suitable signal peptides selected from the mentioned list.

The term "open reading frame" corresponds to a nucleotide sequence in a DNA or RNA molecule that may potentially encode a polypeptide or a protein. Said open reading frame begins with a start codon (the start codon generally encoding a methionine), followed by a series of codons (each codon encoding an amino acid), and ends with a stop codon (the stop codon not being translated).

The term "secretion signal peptide" refers to a highly hydrophobic amino acid sequence (preferably 15 to 60 amino acids long) of proteins that must cross through membranes to arrive at their functioning cellular location. By binding to signal recognition particles, these sequences direct nascent protein-ribosome complexes to a membrane where the protein is inserted during translation. Signal peptides direct translational uptake of the protein by various membranes (e.g. endoplasmic reticulum, mitochondria, chloroplast, peroxisome).

In a preferred embodiment, the signal peptide has the sequence shown in SEQ ID NO: 17. The sequence of PS2Aa1 including the signal peptide has the sequence shown in SEQ ID NO: 2.

MGWSCIILFLVATATGVHSYNDKRMCSDPYQGMNKPHYCNCHTYNNSEITGGLQVDLD
NQVVETFQSTTDVIREYLMFNELSALSSSPESVRSRFSSIYGTNPDGIALNNETYFNAVKP
PITAQYGYYCYKNVGTVQYVNRPTDINPNVILAQDTLTNNTNEPFTTTITITGSFTNTSTVT
SSTTTGFKFTSKLSIKKVFEIGGEVSFSTTIGTSETTTETITVSKSVTVTVPAQSRRTIQLTA
KIAKESADFSAPITVDGYFGANFPKRVGPGGHYFWFNPARDVLNTTSGTLRGTVTNVSS

FDFQTIVQPARSLLDEQQETLEYAIPGDPSGEQLQQMEQRMFFSKCQCPKWGN (SEQ
ID NO: 2)

The recombinant parasporin 2 protein of the invention comprises one heterologous protease cleavage site to allow the removal of the N-terminal sequence that inhibits the pore-forming ability and, optionally, a further heterologous protease cleavage site to allow the removal of the C-terminal sequence that inhibits the pore-forming ability. Therefore in a preferred embodiment, the recombinant parasporin 2 protein of the invention comprises only one heterologous protease cleavage site to allow the removal of the N-terminal sequence. In another preferred embodiment, the recombinant parasporin 2 protein of the invention comprises a heterologous protease cleavage site to allow the removal of the N-terminal sequence and a further heterologous protease cleavage site to allow the removal of the C-terminal sequence that inhibits the pore-forming ability. Said further heterologous cleavage site can be the same or different from the heterologous protease cleavage site to allow the removal of the N-terminal sequence.

"Heterologous", when referring to the protease cleavage site, as used herein, means that the sequence of the protease cleavage site corresponds to a protein different from the parasporin 2.

"Protease cleavage site", as used herein, refers to a polypeptide sequence which can be recognized and processed by a protease when the target site and the protease are contacted under suitable conditions.

Illustrative non-limiting examples of protease cleavage sites are disclosed in Table I.

**Table I. Examples of protease cleavage sites**

| Linker core sequence | SEQ ID NO | Cleaved by |
|---|---|---|
| HSSKLQ | SEQ ID NO:21 | PSA |
| PLGLA | SEQ ID NO:22 | MMPs |
| IGGL | SEQ ID NO:23 | MMPs |
| PXXHy (Hy means hydrophobic residue) | | MMPs |
| LIXXHy (Hy means hydrophobic residue) | | MMP2 |
| HySXL (Hy means hydrophobic residue) | | MMP2 |
| PLGLGL | SEQ ID NO:24 | MMPs |
| PLGMTS | SEQ ID NO:25 | MMPs |
| PLGLWA | SEQ ID NO:26 | MMPs |
| VGR | | uPA |
| LSGRSDNH | SEQ ID NO:27 | uPA, matriptase, leguamin |
| LEVLFQGM | SEQ ID NO: 3 | Human Rhinovirus 3C protease |
| GGSLSGRSDNHGGS | SEQ ID NO: 5 | legumain, uPA and Matriptase |
| SGRS | SEQ ID NO: 7 | trypsin and UPA |
| SGRSG | SEQ ID NO: 8 | trypsin and UPA |
| QRLRR | SEQ ID NO: 10 | furin |
| RRKR | SEQ ID NO: 12 | furin |
| RLRR | SEQ ID NO: 16 | furin |

Protease cleavage sites also suitable for their incorporation into the recombinant parasporin 2 of the invention include enterokinase (cleavage site DDDDK, SEQ ID NO: 28), factor Xa (cleavage site IEDGR, SEQ ID NO:29), thrombin (cleavage site LVPRGS, SEQ ID NO: 30), TEV protease (cleavage site ENLYFQG, SEQ ID NO: 31), PreScission protease (cleavage site LEVLFQGP, SEQ ID NO:32 ), furin ( cleavage consensus motif R-X-R/K/X-R ), inteins and the like.

Additional protease cleavage sites can be found in Choi et al Theranostics 2012, 2 (2), 156-179, Vandooren et al Adv. Drug Deliv. Rev. 2016, 97, 144-155; Dudani et al Annu. Rev. Cancer Biol. 2018, 2 (1), 353-376 or Poreba FEBS J. 2020, 287 (10), 1936-1969.

In a preferred embodiment, the protease cleavage site is the cleavage site of a protease selected from the group consisting of legumain, uPA, matriptase, furin, trypsin and human rhinovirus 3C protease.

"Legumain", also known as asparaginyl endopeptidase or δ-secretase, relates to an endolysosomal cysteine protease. The complete protein sequence for legumain has the UniProt accession number Q99538 (September 13, 2023).

"uPA", also known as urokinase-type plasminogen activator is a serine protease consisting of three domains: the serine protease domain (consisting of residues 159-411), the kringle domain (consisting of residues 50-131), and the EGF-like domain (consisting of residues 1-49). The complete protein sequence for human urokinase-type plasminogen activator has the UniProt accession number P00749 (September 13, 2023), incorporated by reference.

"Matriptase", as used herein, relates to an enzyme family which cleaves various synthetic substrates with Arg or Lys at the P1 position and prefers small side-chain amino acids, such as Ala and Gly, at the P2 position. The complete protein sequence for human matriptase has the UniProt accession number Q8IU80 (September 13, 2023), incorporated by reference.

"Furin", as used herein, relates to a protease, a proteolytic enzyme that in humans and other animals is encoded by the FURIN gene. Furin cleaves proteins just downstream of a basic amino acid target sequence (canonically, Arg-X-(Arg/Lys/X)-Arg'). The complete protein sequence for the human furin has the UniProt accession number P09958 (September 13, 2023), incorporated by reference.

"Trypsin", as used herein, relates to a serine protease enzyme, and hydrolyzes proteins at the carboxyl side of the Lysine or Arginine. The complete protein sequence for the human trypsin has the UniProt accession number P07477 (September 13, 2023), incorporated by reference.

"Human rhinovirus 3C protease" is a recombinant 3C protease derived from human Rhinovirus type 14. The complete protein sequence for the human rhinovirus 3C protease has the UniProt accession number P03300 (May 3, 2023), incorporated by reference.

In a preferred embodiment, the recombinant parasporin 2 protein comprises, consists or consists essentially of the sequence shown in SEQ ID NO: 1, SEQ ID NO: 2 or comprises, consists or consists essentially of a functionally equivalent variant thereof and further comprises one heterologous protease cleavage site to allow the removal of the N-terminal sequence that inhibits the pore-forming ability and, optionally, a further heterologous protease cleavage site to allow the removal of the C-terminal sequence that inhibits the pore-forming ability.

"Functionally equivalent variant" is understood to mean all those polypeptides derived from the sequence SEQ ID NO: 1 or SEQ ID NO: 2 by chemical modification, substitution, insertion and/or removal of one or more amino acids whenever the function is substantially maintained. According to the present invention, the relevant function to be substantially maintained is the pore-forming ability in the absence of the N-terminal and/or C-terminal sequences that inhibits said ability. The ability of the parasporin or variant of forming a pore can be assessed experimentally, for example, by the methods previously mentioned.

In a preferred embodiment, the "functionally equivalent variant" displays an ability to form a pore in the absence of the N-terminal and/or C-terminal sequences that inhibits said ability which is of at least 40%, at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of the ability shown by the sequence SEQ ID NO:1 or SEQ ID NO: 2.

Preferably, "variants" of sequence SEQ ID NO:1 or SEQ ID NO: 2 are (i) polypeptides in which one or more amino acid residues are substituted by a conserved or non-conserved amino acid residue (preferably a preserved amino acid residue) and such substituted amino acid may be coded or not by the genetic code, (ii) polypeptides in which there is one or more modified amino acid residues, for example, residues modified by substituent bonding, (iii) polypeptides resulting from alternative processing of a similar mRNA, (iv) polypeptides fragments and/or (v) polypeptides resulting from the fusion of sequence SEQ ID NO:1 or SEQ ID NO: 2 with itself or of the variants defined above in (i) to (iii) with another polypeptide, such as a secretory leader sequence or a signal peptide, a sequence being used for purification (for example, His tag), for detection (for example, Sv5 epitope tag) or for delivery to a specific target cell. The fragments include polypeptides generated through proteolytic cut (including multisite proteolysis) of an original sequence. The functionally equivalent variant may be the result of a post-translationally or chemically modification. Such variants will be apparent to those skilled in the art.

The term "fragment", as referred to herein, relates to a functional polypeptide derived from SEQ ID NO: 1 or 2 by removal of one or more terminal amino acids at the N-terminus and/or at the C-terminus of an individual SEQ ID NO. Fragments preferably have a length of at least 7 amino acids and/or up to 10%, up to 20%, up to 30%, up to 40%, up to 50%, up to 60%, up to 70%, up to 80%, up to 85%, up to 90%, up to 95%, up to 98%, or up to 99% of the length of SEQ ID NO: 1 or SEQ ID NO: 2.

The "variants" of sequence SEQ ID NO: 1 or SEQ ID NO: 2 may be both natural and artificial. The expression "natural variant" relates to all those variants of SEQ ID NO: 1 or SEQ ID No: 2 which appear naturally in other species, i.e. the orthologues of SEQ ID NO: 1 or SEQ ID NO: 2. Examples of variants of SEQ ID No: 1 or SEQ ID NO: 2 are for example parasporin PS2Aa2 (Cry46Aa2, from strain A1470) having a sequence shown in SEQ ID NO:20 or PS2Ab1 (Cry46Ab from strain TK-E6) having a sequence shown in SEQ ID NO: 14.

In a particular embodiment, the "functionally equivalent variant" of the parasporin 2 of the invention has a sequence which shows at least 70%, at least 80%, at least 85%, at least 90%, at least 95% or at least 99% identity with the sequence of SEQ ID NO:1 or SEQ ID NO: 2. In another preferred embodiment, the "functionally equivalent variant" of the polypeptide of the invention has a sequence which shows at least 75% identity with the sequence of SEQ ID NO: 1 or SEQ ID NO: 2, more preferably at least 80%, at least 85%, at least 90%, at least 91%, at least 92%, at least 93%, at least 94%, at least 95%, at least 96%, at least 97%, at least 98 % or at least 99%.

In a more preferred embodiment, a functionally equivalent variant of SEQ ID NO: 1 is the sequence shown in SEQ ID NO: 14.

SEQ ID NO: 14 corresponds to the sequence of PS2Ab, also PS2Ab1, from B. *thuringiensis* of strain TK-E6 and having a size of 33kDa (304 aa) and is cleaved at its two ends (N and C terminal) by proteinase K, to generate the active form of 29kDa. The sequence of PS2Ab corresponds to the sequence shown in the GenBank database as number AB186914.1 (July 26, 2016) and GenPep database of GenBank BAD35170.1 (July 26, 2016). Wild type PS2Ab has inhibitory domains at both ends of the protein and is cleaved by proteinase K.

In another preferred embodiment, the variant of SEQ ID NO: 1 or SEQ ID NO: 2 is the sequence corresponding to PS2Aa2 shown in SEQ ID NO: 20.

The terms "identity", "identical" or "percent identity", in the context of two or more amino acid or nucleotide sequences, refer to two or more sequences or subsequences that are the same or have a specified percentage of amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. Publicly available software programs can be used to align sequences. Appropriate parameters for maximal alignment by particular alignment software can be determined by one skilled in the art. In certain embodiments, the default parameters of the alignment software are used. In certain embodiments, the percentage identity "X" of a first amino acid sequence to a second amino acid sequence is calculated as 100 x (Y/Z), where Y is the number of amino acid residues scored as identical matches in the alignment of the first and second sequences (as aligned by visual inspection or a particular sequence alignment program) and Z is the total number of residues in the second sequence. If the second sequence is longer than the first sequence, then the global alignment taking the entirety of both sequences into consideration is used, therefore all letters and empty spaces in each sequence must be aligned. In this case, the same formula as above can be used but using as Z value the length of the region wherein the first and second sequence overlaps, said region having a length which is substantially the same as the length of the first sequence.

As a non-limiting example, whether any particular polypeptide has a certain percentage sequence identity (e.g., is at least 80% identical, at least 85% identical, at least 90% identical, and in some embodiments, at least 95%, 96%, 97%, 98%, or 99% identical) to a reference sequence. For instance, 95% identical to a reference sequence according to the present invention, the parameters are set such that the percentage of identity is calculated over the full length of the reference amino acid sequence and that gaps in homology of up to 5% of the total number of nucleotides in the reference sequence are allowed.

In some embodiments, two amino acid sequences are substantially identical, meaning they have at least 70%, at least 75%, at least 80%, at least 85%, at least 90%, and in some embodiments at least 95%, 96%, 97%, 98%, 99% amino acid residue identity, when compared and aligned for maximum correspondence, as measured using a sequence comparison algorithm or by visual inspection. Identity can exist over a region of the sequences that is at least about 10, about 20, about 40-60 residues in length or any integral value there between, and can be over a longer region than 60-80 residues, for example, at least about 90-100 residues, and in some embodiments, the sequences are substantially identical over the full length of the sequences being compared.

The recombinant parasporin 2 protein comprises one heterologous protease cleavage site to allow the removal of the N-terminal sequence that inhibits the pore-forming ability and, optionally, a further heterologous protease cleavage site to allow the removal of the C-terminal sequence that inhibits the pore-forming ability.

In a preferred embodiment, the heterologous protease cleavage site is inserted in a location after the N-terminal sequence that inhibits the pore-forming ability and/or before the C-terminal sequence that inhibits the pore-forming ability.

In another preferred embodiment, the location to substitute or insert a protease cleavage site is shown in brackets. This means that a heterologous protease cleavage site can be inserted after any of the residues located in brackets or replacing any of the residues located in brackets in SEQ ID NO: 1.

MYNDKRMCSD PYQGMNKPHY CNCHTYNNSE ITGGLQVDLD 40

NQVVETFQST TDVIREYLMF NELSALSSSP ESVRSRFSSI 80

YGTNPDGIAL NNETYFNAVK PPITAQYGYY CYKNVGTVQY 120

VNRPTDINPN VILAQDTLTN NTNEPFTTTI TITGSFTNTS 160

TVTSSTTTGF KFTSKLSIKK VFEIGGEVSF STTIGTSETT 200

TETITVSKSV TVTVPAQSRR TIQLTAKIAK ESADFSAPIT 240

VDGYFGANFP KRVGPGGHYF WFNPARDVLN TTSGTLRGTV 280

TNVSSFDFQT IVQPARSLLD EQQETLEYAI PGDPSGEQLQ 320

QMEQRMFFSK CQCPKWGN* 338

Therefore, in a preferred embodiment, a protease cleavage site is inserted after S49, after T50, after T51, after D52, and/or after V53 of SEQ ID NO: 1. In a preferred embodiment, the protease cleavage site is inserted between S49 and T50 of SEQ ID NO: 1. In another preferred embodiment, the protease cleavage site is inserted between T50 and T51 of SEQ ID NO: 1. In another preferred embodiment, the protease cleavage site is inserted between T51 and D52 of SEQ ID NO: 1. In another preferred embodiment, the protease cleavage site is inserted between D52 and V53 of SEQ ID NO: 1. In another preferred embodiment, the protease cleavage site is inserted between V53 and I54 of SEQ ID NO: 1.

In another preferred embodiment, a heterologous protease cleavage site is inserted by substituting any of the residues selected from the group consisting of S49, T50, T51, D52 and V53. In another preferred embodiment, a protease cleavage site is inserted by substituting the residues from S49 to T50, from S49 to T51, from S49 to D52, from S49 to V53, from T50 to T51, from T50 to D52, from T50 to V53, from T51 to D52, from T51 to V53, or from D52 to V53 all referring to SEQ ID NO: 1.

In another preferred embodiment, the further protease cleavage site that allows the removal of the C-terminal sequence that inhibits the pore-forming ability is inserted after L298, after L299, after D300, after E301, after Q302, after Q303, and/or after E304 of SEQ ID NO: 1. In a preferred embodiment, the protease cleavage site is inserted between L298 and L299 of SEQ ID NO: 1. In a preferred embodiment, the protease cleavage site is inserted between L299 and D300 of SEQ ID NO: 1. In a preferred embodiment, the protease cleavage site is inserted between D300 and E301 of SEQ ID NO: 1. In a preferred embodiment, the protease cleavage site is inserted between E301 and Q302 of SEQ ID NO: 1. In a preferred embodiment, the protease cleavage site is inserted between Q302 and Q303 of SEQ ID NO: 1. In a preferred embodiment, the protease cleavage site is inserted between Q303 and E304 of SEQ ID NO: 1.

In another preferred embodiment, the further heterologous protease cleavage site is inserted by substituting any of the residues selected from the group consisting of L298, L299, D300, E301, Q302, Q303 and E304. In another preferred embodiment, a protease cleavage site is inserted by substituting the residues from L298 to L299, from L298 to D300 from L298 to E301, from L298 to Q302, from L298 to Q303, or from L298 to E304 all referring to SEQ ID NO: 1. In another preferred embodiment, the protease cleavage site is inserted by substituting the residues from L299 to D300, from L299 to E301, from L299 to Q302, from L299 to Q303, or from L299 to E304 all referring to SEQ ID No: 1. In another preferred embodiment, the protease cleavage site is inserted by substituting the residues from D300 to E301, from D300 to Q302 from D300 to Q303, or from D300 to E304 all referring to SEQ ID No: 1. In another preferred embodiment, the protease cleavage site is inserted by substituting the residues from E301 to Q302 , from E301 to Q303, or from E301 to E304 all referring to SEQ ID NO: 1. In another preferred embodiment, the protease cleavage site is inserted by substituting the residues from Q302 to Q303 or from Q302 to E304 of SEQ ID NO: 1. In another preferred embodiment, the further heterologous protease cleavage site is inserted by substituting the residues from Q303 to E304 of SEQ ID NO: 1. In another preferred embodiment, the protease cleavage site is inserted by substituting the residues form E304 to T305 of SEQ ID NO: 1.

In the case that the functionally equivalent variant of SEQ ID NO: 1 is the sequence shown in SEQ ID NO: 20, preferably a heterologous protease cleavage site is inserted after S49, after T50, after T51, after D52, or after V53 of SEQ ID NO: 20. In a preferred embodiment, the protease cleavage site is inserted between S49 and T50 of SEQ ID NO: 20. In another preferred embodiment, the protease cleavage site is inserted between T50 and T51 of SEQ ID NO: 20. In another preferred embodiment, the protease cleavage site is inserted between T51 and D52 of SEQ ID NO: 20. In another preferred embodiment, the protease cleavage site is inserted between D52 and V53 of SEQ ID NO: 20. In another preferred embodiment, the protease cleavage site is inserted between V53 and I54 of SEQ ID NO: 20.

In another preferred embodiment, a heterologous protease cleavage site is inserted by substituting any of the residues selected from the group consisting of S49, T50, T51, D52 and V53 of SEQ ID NO: 20. In another preferred embodiment, a protease cleavage site is inserted by substituting the residues from S49 to T50, from S49 to T51, from S49 to D52, from S49 to V53, from T50 to T51, from T50 to D52, from T50 to V53, from T51 to D52, from T51 to V53, or from D52 to V53 all referring to SEQ ID NO: 20.

In another preferred embodiment, the further protease cleavage site that allows the removal of the C-terminal sequence that inhibits the pore-forming ability is inserted after L298, after L299, after D300, after E301, after Q302, and/ or after Q303 of SEQ ID NO: 20. In a preferred embodiment, the protease cleavage site is inserted between S49 and T50 of SEQ ID NO: 20. In another preferred embodiment, the protease cleavage site is inserted between T50 and T51 of SEQ ID NO: 20. In another preferred embodiment, the protease cleavage site is inserted between T51 and D52 of SEQ ID NO: 20. In another preferred embodiment, the protease cleavage site is inserted between D52 and V53 of SEQ ID NO: 20. In another preferred embodiment, the protease cleavage site is inserted between V53 and I54 of SEQ ID NO: 20.

In another preferred embodiment, a heterologous protease cleavage site is inserted by substituting any of the residues selected from the group consisting of L298, L299, D300, E301, Q302, Q303, and E304. In another preferred embodiment, a protease cleavage site is inserted by substituting the residues from L298 to L299, from L298 to D300, from L298 to E301, from L298 to Q302, from L298 to Q303, or from L298 to E304 all referring to SEQ ID NO: 20. In another preferred embodiment, the protease cleavage site is inserted by substituting the residues from L299 to D300, from L299 to E301, from L299 to Q302, from L299 to Q303, or from L200 to E304 all referring to SEQ ID NO: 20. In another preferred embodiment, the protease cleavage site is inserted by substituting the residues from D300 to E301, from D300 to Q302, from D300 to Q303, or from D300 to E304 all referring to SEQ ID NO: 20. In another preferred embodiment, the protease cleavage site is inserted by substituting the residues from E301 to Q302, from E301 to Q303, or from E301 to E304 all referring to SEQ ID NO: 20. In another preferred embodiment, the protease cleavage site is inserted by substituting the residues from Q302 to Q303, from Q302 to E304, or from Q302 to E304 of SEQ ID NO: 20. In another preferred embodiment, the protease cleavage site is inserted by substituting the residues form Q303 to E304 of SEQ ID NO: 20. In another preferred embodiment, the protease cleavage site is inserted by substituting the residues form E304 to T305 of SEQ ID NO: 20.

In the case that the functionally equivalent variant of SEQ ID NO: 1 is the sequence shown in SEQ ID NO: 14, a heterologous protease cleavage site is inserted after P25, after S26, after T27, after N28, or after V29 of SEQ ID NO: 14. In a preferred embodiment, the protease cleavage site is inserted between P25 and S26 of SEQ ID NO: 14. In a preferred embodiment, the protease cleavage site is inserted between S26 and T27 of SEQ ID NO: 14. In a preferred embodiment, the protease cleavage site is inserted between T27 and N28 of SEQ ID NO: 14. In a preferred embodiment, the protease cleavage site is inserted between N28 and V29 of SEQ ID NO: 14. In a preferred embodiment, the protease cleavage site is inserted between V29 and I30 of SEQ ID NO: 14.

In another preferred embodiment, a heterologous protease cleavage site is inserted by substituting any of the residues selected from the group consisting of P25, S26, T27, N28, or V29 of SEQ ID NO: 14. In another preferred embodiment, a protease cleavage site is inserted by substituting the residues from P25 to S26, from P25 to T27, from P25 to N28, or from P25 to V29 of SEQ ID NO: 14. In another preferred embodiment, the heterologous protease cleavage site is inserted by substituting the residues from S26 to T27, from S26 to N28, from S26 to V29, from T27 to N28, or from T27 to V29, all referring to SEQ ID NO: 14. In another preferred embodiment, the heterologous protease cleavage site is inserted by substituting the residues from N28 to V29 of SEQ ID NO. 14. In another preferred embodiment, the heterologous protease cleavage site is inserted by substituting the residues from V29 to I30 of SEQ ID NO: 14.

In another preferred embodiment, the further protease cleavage site that allows the removal of the C-terminal sequence that inhibits the pore-forming ability is inserted after L274, after L275, after A276, after E277, after Q278, after Q279, or after E280. In another preferred embodiment, a heterologous protease cleavage site is inserted by substituting any of the residues selected from the group consisting of L274, L275, A276, E277, Q278, Q279 and E280. In another preferred embodiment, a protease cleavage site is inserted by substituting the residues from L274 to L275, from L274 to A276, from L274 to E277, from L274 to Q278, from L274 to Q279, or from L274 to E280 all referring to SEQ ID NO: 14. In another preferred embodiment, the protease cleavage site is inserted by substituting the residues from L275 to A276, from L275 to E277, from L275 to Q278, from L275 to Q279, or from L2275 to E280 all referring to SEQ ID NO: 14. In another preferred embodiment, the protease cleavage site is inserted by substituting the residues from A276 to E277, from A276 to Q278, from A276 to Q279, or from A276 to E280 all referring to SEQ ID NO: 14. In another preferred embodiment, the protease cleavage site is inserted by substituting the residues from E277 to Q278, from E277 to Q279, or from E277 to E380 of SEQ ID NO: 14. In another preferred embodiment, the further protease cleavage site is inserted by substituting the residues from Q278 to Q279 or from Q278 to E280 of SEQ ID NO: 14. In another preferred embodiment, the further protease cleavage site is inserted by substituting the residues from Q279 to E280 of SEQ ID NO: 14.

In another preferred embodiment, a heterologous protease cleavage site is inserted after one of the residues selected from the group consisting of T51, Q302 and/or Q303 of SEQ ID NO: 1, which means between T51 and D52, between Q302 and Q303, and between Q303 and E304, respectively. In a more preferred embodiment, the heterologous protease cleavage site is inserted after T51 of SEQ ID NO: 1. In another preferred embodiment, a heterologous protease cleavage site is inserted after the residue Q302 of SEQ ID NO: 1 In another preferred embodiment, the residue T51 or the residues from D300 to E304, from T50 to D52, and/or from T51 to D52 of SEQ ID NO: 1 have been replaced by a protease cleavage site.

In a more preferred embodiment, the recombinant parasporin 2 protein comprises a protease cleavage site after T51 and after Q302 of SEQ ID NO: 1, preferably the protease cleavage site is the sequence shown in SEQ ID NO: 3. In a preferred embodiment, the recombinant parasporin 2 protein comprises, consists or consists essentially of the sequence shown in SEQ ID NO: 4. This sequence corresponds to the protein HPS2Aa1 of the invention.

In another more preferred embodiment, the recombinant parasporin 2 protein comprises a protease cleavage site after T51 and after Q302 of SEQ ID NO: 1, preferably between the protease cleavage site of sequence shown in SEQ ID NO: 5. In a preferred embodiment, the recombinant parasporin 2 protein comprises, consists or consists essentially of the sequence shown in SEQ ID NO: 6. This sequence corresponds to the protein PS2Aa1-3 of the invention.

In another preferred embodiment, the residues from T50 to D52 and from D300 to E304 in SEQ ID NO: 1 have been replaced by a protease cleavage site. Preferably, the protease cleavage site replacing the residues from T50 to D52 is the sequence shown in SEQ ID NO: 7. In another preferred embodiment, the protease cleavage site replacing the residues from D300 to E304 is the sequence shown in SEQ ID NO: 8.

In a more preferred embodiment, the recombinant parasporin 2 protein comprises, consists or consists essentially of the sequence shown in SEQ ID NO: 9. This sequence corresponds to PS2Aa1-1 of the invention.

In another preferred embodiment, the residues from T51 to D52 of parasporin 2 have been replaced by the protease cleavage site sequence shown in SEQ ID NO: 10. More preferably the recombinant parasporin 2 protein comprises, consists or consists essentially of the sequence shown in SEQ ID NO: 11. This sequence corresponds to PS2Aa1-Fu of the invention.

In another preferred embodiment, the recombinant parasporin 2 of the invention, the residue T51 has been replaced by the cleavage site sequence shown in SEQ ID NO: 10 and further comprises a cleavage site sequence shown in SEQ ID NO: 12 after residue Q303. More preferably, the recombinant parasporin 2 protein comprises, consists or consists essentially of the sequence shown in SEQ ID NO: 13. This sequence corresponds to PS2Aa1-Fu2 of the invention.

In another preferred embodiment, the recombinant parasporin 2 protein according to the invention having the sequence shown in SEQ ID NO: 14 comprises a protease cleavage site after T27 and optionally a further protease cleavage site after Q279. In a more preferred embodiment, the protease cleavage site after T27 has the sequence shown in SEQ ID NO: 16. In another preferred embodiment, the protease cleavage site after Q279 has the sequence shown in SEQ ID NO. 12. In a more preferred embodiment, the recombinant parasporin 2 according to the invention comprises, consists or consists essentially of the sequence shown in SEQ ID NO: 15. This sequence corresponds to PS2Ab-Fu2 of the invention.

In another particular embodiment, the parasporin 2 of the invention has a sequence which shows at least 70%, at least 75 %, at least 80%, at least 85%, at least 90%, at least 95% or at least 99% identity with the sequence of SEQ ID NO: 4, SEQ ID NO: 6, SEQ ID NO: 9, SEQ ID NO: 11, SEQ ID NO: 13, SEQ ID NO: 14 or SEQ ID NO: 15.

In another aspect, the invention relates to a nucleic acid encoding a recombinant parasporin 2 protein according to the invention.

As used herein, the term "nucleic acid or polynucleotide" refers to a polymer composed of a multiplicity of nucleotide units (deoxyribonucleotides or ribonucleotides, or related structural variants or synthetic analogues thereof) linked via phosphodiester bonds (or related structural variants or synthetic analogues thereof). The term polynucleotide includes double or single stranded genomic and cDNA, RNA, any synthetic and genetically manipulated polynucleotide, and both sense and anti-sense polynucleotide (although only sense strands are being disclosed in the present invention). This includes single- and double-stranded molecules, i.e., DNA-DNA, DNA-RNA and RNA-RNA hybrids.

Recombinant techniques may be used to generate the nucleic acid of the present invention. To produce a polypeptide of the present invention using recombinant technology, a polynucleotide encoding the polypeptide of the present invention is ligated into a nucleic acid expression vector, which comprises the polynucleotide sequence under the transcriptional control of a cis-regulatory sequence (e.g., promoter sequence) suitable for directing constitutive, species specific, cell specific, tissue specific or inducible transcription of the monomers, dimers or trimers of the present invention in the host cells.

In addition to being synthesizable in host cells, the polypeptides of the present invention can also be synthesized using *in vitro* expression systems. These methods are well known in the art and the components of the system are commercially available.

In another aspect, the invention relates to an expression cassette comprising the nucleic acid according to the invention.

The term "expression cassette", as used herein, refers to a distinct component of DNA which comprises a gene and a regulatory sequence, such as a promoter, which allows the expression cassette to produce RNA and protein when inserted into the desired host cell. More preferably, the expression cassette of the invention comprises a promoter sequence. The optional selection marker is an antibiotic resistance gene (e.g. kanamycin, ampicillin, tetracycline, spectinomycin), preferably ampicillin.

In another aspect, the invention relates to a vector comprising the expression cassette according to the invention.

The term "vector", as used herein, refers to a construct capable of delivering, and preferably additionally expressing, one or more polynucleotides of interest into a host cell. Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. This term also relates to targeting constructs, which allow for random or site-directed integration of the targeting construct into genomic DNA. Such targeting constructs, preferably, comprise DNA of sufficient length for either homologous recombination or heterologous integration. In a particular embodiment, the vector is an expression vector. The term "expression vector" refers to a replicative DNA construct used for expressing the nucleic acid construct of the invention in a cell, preferably a eukaryotic cell, more preferably a mammalian cell. The expression vector also preferably contains an origin of replication in prokaryotes, necessary for vector propagation in bacteria. Additionally, the expression vector can also contain a selection gene for bacteria, for example, a gene encoding a protein conferring resistance to an antibiotic, for example, ampicillin, kanamycin, chloramphenicol, etc. The expression vector can also contain one or more multiple cloning sites.

Vectors suitable for the insertion of said polynucleotide are vectors derived from expression vectors in prokaryotes such as pUC18, pUC19, Bluescript and the derivatives thereof, mpl8, mpl9, pBR322, pMB9, ColEl, pCRI, RP4, phages and "shuttle" vectors such as pSA3 and pAT28; expression vectors in yeasts such as vectors of the type of 2 micron plasmids, integration plasmids, YEP vectors, centromere plasmids and the like; expression vectors in insect cells such as vectors of the pAC and pVL; expression vectors in plants such as pIBI, pEarleyGate, pAVA, pCAMBIA, pGSA, pGWB, pMDC, pMY, pORE series and the like; and expression vectors in eukaryotic cells, including baculovirus suitable for transfecting insect cells using any commercially available baculovirus system such as pFastBac1. The vectors for eukaryotic cells include, preferably, viral vectors (adenoviruses, viruses associated to adenoviruses such as retroviruses and, particularly, lentiviruses) as well as non-viral vectors such as pSilencer 4.1-CMV (Ambion), pcDNA3, pcDNA3.1/hyg, pHMCV/Zeo, pCR3.1, pEFI/His, pIND/GS, pRc/HCMV2, pSV40/Zeo2, pTRACER-HCMV, pUB6/V5-His, pVAXI, pZeoSV2, pCI, pSVL and PKSV-10, pBPV-1, pML2d and pTDTI .

The vectors may also comprise a reporter or marker gene which allows identifying those cells that have incorporated the vector after having been put in contact with it.

Useful reporter genes in the context of the present invention include lacZ, luciferase, thymidine kinase, GFP and the like. Useful marker genes in the context of this invention include, for example, the neomycin resistance gene, conferring resistance to the aminoglycoside G418; the hygromycin phosphotransferase gene, conferring resistance to hygromycin; the ODC gene, conferring resistance to the inhibitor of the ornithine decarboxylase (2-(difluoromethyl)-DL-ornithine (DFMO); the dihydrofolatereductase gene, conferring resistance to methotrexate; the puromycin-N-acetyl transferase gene, conferring resistance to puromycin; the ble gene, conferring resistance to zeocin; the adenosine deaminase gene, conferring resistance to 9-beta-D-xylofuranose adenine; the cytosine deaminase gene, allowing the cells to grow in the presence of N-(phosphonacetyl)-L-aspartate; thymidine kinase, allowing the cells to grow in the presence of aminopterin; the xanthine-guanine phosphoribosyltransferase gene, allowing the cells to grow in the presence of xanthine and the absence of guanine; the trpB gene of *E*. *coli*, allowing the cells to grow in the presence of indole instead of tryptophan; the hisD gene of *E.coli,* allowing the cells to use histidinol instead of histidine. The selection gene is incorporated into a plasmid that can additionally include a promoter suitable for the expression of said gene in eukaryotic cells (for example, the CMV or SV40 promoters), an optimized translation initiation site (for example, a site following the so-called Kozak's rules or an IRES), a polyadenylation site such as, for example, the SV40 polyadenylation or phosphoglycerate kinase site, introns such as, for example, the beta-globulin gene intron. Alternatively, it is possible to use a combination of both the reporter gene and the marker gene simultaneously in the same vector. In a preferred embodiment, the promoter is CMV.

On the other hand, as the person skilled in the art is aware, the choice of the vector will depend on the host cell in which it will subsequently be introduced. By way of example, the vector in which said polynucleotide is introduced can also be a yeast artificial chromosome (YAC), a bacterial artificial chromosome (BAC) or a PI -derived artificial chromosome (PAC). The characteristics of the YAC, BAC and PAC are known by the person skilled in the art. The vector of the invention can be obtained by conventional methods known by persons skilled in the art.

The polynucleotide of the invention can be introduced into the host cell *in vivo* as naked DNA plasmids, but also using vectors by methods known in the art, including but not limited to transfection, electroporation (e.g. transcutaneous electroporation), microinjection, transduction, cell fusion, DEAE dextran, calcium phosphate precipitation, use of a gene gun, or use of a DNA vector transporter. In a preferred embodiment, the polynucleotide is introduced into the host cell by calcium phosphate HBS method. Methods for formulating and administering naked DNA to mammalian muscle tissue are also known. Other molecules are also useful for facilitating transfection of a nucleic acid *in vivo,* such as cationic oligopeptides, peptides derived from DNA binding proteins, or cationic polymers.

Another well-known method that can be used to introduce polynucleotides into host cells is particle bombardment (aka biolistic transformation). Biolistic transformation is commonly accomplished in one of several ways. One common method involves propelling inert or biologically active particles at cells.

Alternatively, the vector can be introduced *in vivo* by lipofection. The use of cationic lipids can promote encapsulation of negatively charged nucleic acids, and also promote fusion with negatively charged cell membranes. Particularly useful lipid compounds and compositions for transfer of nucleic acids have been described.

In a particular embodiment, the expression vector is a viral vector or virus comprising the nucleic acid of the invention, or the expression vector of the invention. Thus, in another embodiment, the present invention relates to a virus containing the nucleic acid of the second aspect. Suitable viruses are safe, have low toxicity and are genetically stable. Non-limiting examples are retroviruses, particularly lentiviruses, adenoviruses and adeno-associated viruses (AAVs). In a more particular embodiment, the expression vector is a lentiviral vector or an adenoviral vector; more preferably an adenoviral vector. The term "lentiviral vector", as used herein, refers to a vector based on a group (or scientific genus) of retroviruses that in nature give rise to slowly developing disease due to their ability to incorporate into a host genome. Modified lentiviral genomes are useful as viral vectors for the delivery of a nucleic acid sequence to a cell. An advantage of lentiviruses for infection of cells is the ability for sustained transgene expression. These viruses include, in particular, Human Immunodeficiency Virus type 1 (HIV-1), Human Immunodeficiency Virus type 2 (HIV-2), Simian Immunodeficiency Virus (SIV), Feline Immunodeficiency Virus (FIV), Equine Infectious Anaemia Virus (EIAV), Bovine Immunodeficiency Virus (BIV), Visna Virus of sheep (VISNA), and Caprine Arthritis-Encephalitis Virus (CAEV). Recombinant lentiviral vectors are capable of infecting non-dividing cells and can be used for both *in vivo* and ex *vivo* gene transfer and expression of nucleic acid sequences. For example, recombinant lentivirus capable of infecting a non-dividing cell wherein a suitable host cell is transfected with two or more vectors carrying the packaging functions, namely gag, pol and env, as well as rev and tat, is described in U.S. Pat. No. 5,994,136. One may target the recombinant virus by linkage of the envelope protein with an antibody or a particular ligand for targeting to a receptor of a particular cell-type. By inserting a sequence (including a regulatory region) of interest into the viral vector, along with another gene which encodes the ligand for a receptor on a specific target cell, for example, the vector is now target-specific. The lentiviral vectors according to the invention may be genetically modified in such a way that certain genes constituting the native infectious virus are eliminated and replaced with a nucleic acid sequence of interest to be introduced into the target cells. The term "adenoviral vector", as used herein, refers to a vector based on an adenovirus. The term "adenovirus" refers to any virus pertaining to the Adenoviridae family characterized by being a non-enveloped virus with a pseudo-icosahedral nucleocapsid containing a double stranded DNA genome. This term includes any adenovirus capable of infecting a human or an animal, including all groups, subgroups, and serotypes that use CAR, CD46 or desmoglein-2 as receptor for infection of target cells. The term adenovirus includes, without limitation, avian, canine, equine, bovine, ovine, porcine, human or frog adenovirus. In a particular embodiment, the adenovirus is a human adenovirus, i.e. an adenovirus capable of infecting humans. A "serotype" is each of the immunologically different types of adenovirus. There are at least 57 serotypes of human adenovirus that are classified into several subgroups (A to G). In another embodiment, the viral vector is a vector based on a virus or the Parvoviridae family, preferably from the *Parvovirinae* subfamily, more preferably from the *Dependoparvovirus* genus, and yet even more preferably an adeno-associated virus.

In a preferred embodiment, the vector is a viral vector, particularly an oncolytic viral vector, more particularly an oncolytic adenoviral vector.

"Oncolytic virus" or "oncolytic viral vector", as used herein, relates to a virus that infects and lyses cancer cells but not normal cells.

In another aspect, the invention relates to a cell comprising a vector according to the invention.

The term "host cell", as used herein, refers to a cell into which a nucleic acid of the invention, such as a polynucleotide or a vector according to the invention, has been introduced and is capable of expressing the polypeptides of the invention as monomers, dimers or trimers or a combination thereof. The terms "host cell" and "recombinant host cell" are used interchangeably herein. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein. The term includes any cultivatable cell that can be modified by the introduction of heterologous DNA. Preferably, a host cell is one in which the polynucleotide of the invention can be stably expressed, post-translationally modified, localized to the appropriate subcellular compartment, and made to engage the appropriate transcription machinery. The choice of an appropriate host cell will also be influenced by the choice of detection signal. For example, reporter constructs, as described above, can provide a selectable or screenable trait upon activation or inhibition of gene transcription in response to a transcriptional regulatory protein; in order to achieve optimal selection or screening, the host cell phenotype will be considered. A host cell of the present invention includes prokaryotic cells and eukaryotic cells. Prokaryotes include gram-negative or gram-positive bacteria, for example, *E. coli* or *Bacilli.* It is to be understood that prokaryotic cells will be used, preferably, for the propagation of the transcription control sequence comprising polynucleotides or the vector of the present invention. Suitable prokaryotic host cells for transformation include, for example, *E. coli, Bacillus subtilis, Salmonella typhimurium,* and various other species within the genera *Pseudomonas, Streptomyces,* and *Staphylococcus.* Eukaryotic cells include, but are not limited to, yeast cells, plant cells, fungal cells, insect cells (e.g., baculovirus), mammalian cells, and the cells of parasitic organisms, e.g., tηpanosomes. As used herein, yeast includes not only yeast in a strict taxonomic sense, i.e., unicellular organisms, but also yeast-like multicellular fungi of filamentous fungi. Exemplary species include *Kluyverei lactis, Schizosaccharomyces pombe,* and *Ustilaqo maydis,* with *Saccharomyces cerevisiae* being preferred. Other yeasts which can be used in practicing the present invention are *Neurospora crassa, Aspergillus niger, Aspergillus nidulans, Pichia pastoris, Candida tropicalis,* and *Hansenula polymorpha.* Mammalian host cell culture systems include established cell lines such as COS cells, L cells, 3T3 cells, Chinese hamster ovary (CHO) cells, embryonic stem cells, with BHK, HeK or HeLa, insect cells, for example but not restricted to, BTI-Tn-5B1-4 (Thermo Fisher Scientific) being preferred. Eukaryotic cells are, preferably, used for recombinant gene expression by applying the transcription control sequence or the expression vector of the present invention.

All the terms and embodiments described elsewhere herein are equally applicable to these aspects of the invention.

### Pharmaceutical composition and therapeutic uses

In another aspect, the invention relates to a pharmaceutical composition comprising a recombinant parasporin 2 protein according to the invention, a nucleic acid according to the invention, an expression cassette according to the invention, a vector according to the invention or a cell according to the invention and a pharmaceutically acceptable carrier or excipient. In a preferred embodiment, the pharmaceutical composition comprises a nucleic acid according to the invention. In a preferred embodiment, the pharmaceutical composition comprises an expression cassette according to the invention. In a preferred embodiment, the pharmaceutical composition comprises a vector according to the invention.

The term "pharmaceutical composition", as used herein, refers to a composition comprising a therapeutically effective amount of the peptide or the nucleic acid construct according to the present invention (or the vector, viral particle or cell comprising said nucleic acid construct) and at least one pharmaceutically acceptable excipient. Pharmaceutical compositions according to the invention can be prepared, for instance, as injectables such as liquid solutions, suspensions, and emulsions. The terms "pharmaceutically acceptable excipient", or "pharmaceutically acceptable carrier," "pharmaceutically acceptable diluent", or "pharmaceutically acceptable vehicle," used interchangeably herein, refer to a non-toxic solid, semisolid or liquid filler, diluent, encapsulating material or formulation auxiliary of any conventional type. A pharmaceutically acceptable carrier is essentially non-toxic to recipients at the dosages and concentrations employed, and is compatible with other ingredients of the formulation. Suitable carriers include, but are not limited to water, dextrose, glycerol, saline, ethanol, and combinations thereof. The carrier can contain additional agents such as wetting or emulsifying agents, pH buffering agents, or adjuvants which enhance the effectiveness of the formulation. Adjuvants could be selected from the group consisting of sterile liquids, such as water and oils, including those of petroleum, animal, vegetable, or synthetic origin, such as peanut oil, soybean oil, mineral oil, sesame oil, and the like. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005.

As used herein, "pharmaceutically acceptable carrier or excipient" can refer to a carrier or excipient that is useful in preparing a pharmaceutical formulation that is generally safe, non-toxic, and is neither biologically or otherwise undesirable, and includes a carrier or excipient that is acceptable for veterinary use as well as human pharmaceutical use. A "pharmaceutically acceptable carrier or excipient" as used in the specification and claims includes both one and more than one such carrier or excipient.

The recombinant parasporin 2 protein according to the invention, the nucleic acid according to the invention, the expression cassette according to the invention, the vector according to the invention or the cell according to the invention are present in a therapeutically effective amount in the pharmaceutical composition of the invention.

The term "therapeutically effective amount", as used herein, in relation to the compound of the invention, or in relation to the compound, excipient and/or carrier comprised by the pharmaceutical composition of the invention, relates to the sufficient amount of said compound, excipient and/or carrier to provide the desired effect, i.e. to achieve an appreciable prevention, cure, delay, reduction of severity or amelioration of one or more symptoms derived from a disease, and will generally be determined by, among other causes, the characteristics of the agent itself and the therapeutic effect to be achieved. It will also depend on the subject to be treated, the severity of the disease suffered by said subject, the chosen dosage form, etc. For this reason, the doses that may be mentioned in this invention must be considered only as guides for the person skilled in the art, who must adjust the doses depending on the aforementioned variables. In an embodiment, the effective amount produces the amelioration of one or more symptoms of the disease that is being treated. Even though individual needs vary, determination of optimal ranges for therapeutically effective amounts of the compounds according to the invention belongs to the common experience of those experts in the art. In general, the dosage needed to provide an effective treatment, which can be adjusted by one expert in the art, will vary depending on age, health, fitness, sex, diet, weight, degree of alteration of the biological target, frequency of treatment, nature and condition of the injury, nature and extent of impairment or illness, medical condition of the subject, route of administration, pharmacological considerations such as activity, efficacy, pharmacokinetic and toxicology profile of the particular compound used, if using a system drug delivery, and if the compound is administered as part of a combination of drugs. The amount of the compound according to the invention that is therapeutically effective in the prevention and/or treatment of cancer in a subject can be determined by conventional clinical techniques (see, for example, The Physician's Desk Reference, Medical Economics Company, Inc., Oradell, NJ, 1995, and Drug Facts and Comparisons, Inc., St. Louis, MO, 1993).

The pharmaceutical composition of the invention can comprise more than one recombinant parasporin 2, nucleic acid, vector or cell of the invention.

In another aspect, the invention relates to a recombinant parasporin 2 according to the invention, a nucleic acid according to the invention, an expression cassette according to the invention, a vector according to the invention, a cell according to the invention or a pharmaceutical composition according to the invention for use in medicine.

In another aspect, the invention relates to a recombinant parasporin 2 according to the invention, a nucleic acid according to the invention, an expression cassette according to the invention, a vector according to the invention, a cell according to the invention or a pharmaceutical composition according to the invention for use in the prevention and/or treatment of cancer.

In another aspect, the invention relates to the use of a recombinant parasporin 2 according to the invention, a nucleic acid according to the invention, an expression cassette according to the invention, a vector according to the invention, or a cell according to the invention for the preparation of a medicament for the prevention and/or treatment of cancer.

Alternatively, the invention relates to a method of preventing and/or treating cancer in a subject comprising administering the recombinant parasporin 2 according to the invention, a nucleic acid according to the invention, an expression cassette according to the invention, a vector according to the invention, a cell according to the invention or a pharmaceutical composition according to the invention to a subject in need thereof.

The term "treat" or "treatment" is used to designate the administration of a composition of the invention or of a medicament containing it to control the progression of the disease before or after clinical signs have appeared. Control of the disease progression is understood to mean the beneficial or desired clinical results that include, but are not limited to, reduction of the symptoms, reduction of the duration of the disease, stabilization of pathological states (specifically to avoid additional deterioration), delaying the progression of the disease, improving the pathological state and remission (both partial and total). The control of progression of the disease also involves an extension of survival, compared with the expected survival if treatment was not applied. Thus, "treatment," "treating," and their equivalent terms refer to obtaining a desired pharmacologic or physiologic effect, covering any treatment of a pathological condition or disorder in a mammal. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or, at least, symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter.

The terms "prevent," "preventing," and "prevention", as used herein, refer to inhibiting the inception or decreasing the occurrence of a disease in an animal. Prevention may be complete (e.g. the total absence of pathological cells in a subject). The prevention may also be partial, such that for example the occurrence of pathological cells in a subject is less than that which would have occurred without the present invention. Prevention also refers to reduced susceptibility to a clinical condition.

The term "cancer", as used herein, refers to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body.

Examples of cancer or tumor include, without limitation, breast, heart, lung, small, intestine, colon, spleen, kidney, bladder, head, neck, ovarian, prostate, brain, rectum, pancreas, skin, bone, bone marrow, blood, thymus, uterus, testicles, hepatobiliary and liver tumors. In particular, the tumor/cancer can be selected from the group of adenoma, angiosarcoma, astrocytoma, epithelial carcinoma, germinoma, glioblastoma, glioma, hemangioendothelioma, hepatoblastoma, leukemia, lymphoma, medulloblastoma, melanoma, neuroblastoma, hepatobiliary cancer, osteosarcoma, retinoblastoma, rhabdomyosarcoma, sarcoma, teratoma, acrallentiginous melanoma, actinic keratosis adenocarcinoma, adenoid cystic carcinoma, adenosarcoma, adenosquamous carcinoma, astrocytic tumors, bartholin gland carcinoma, basal cell carcinoma, bronchial gland carcinoma, carcinosarcoma, cholangiocarcinoma, cystadenoma, endodermal sinus tumor, endometrial hyperplasia, endometrial stromal sarcoma, endometrioid adenocarcinoma, ependymal sarcoma, Swing's sarcoma, focal nodular hyperplasia, germ cell tumors, glucagonoma, hemangioblastoma, hemangioma, hepatic adenoma, hepatic adenomatosis, hepatocellular carcinoma, insulinoma, intraepithelial neoplasia, interepithelial squamous cell neoplasia, invasive squamous cell carcinoma, large cell carcinoma, leiomyosarcoma, malignant melanoma, malignant mesothelial tumor, medulloepithelioma, mucoepidermoid carcinoma, neuroepithelial adenocarcinoma, nodular melanoma, papillary serous adenocarcinoma, pituitary tumors, plasmacytoma, pseudosarcoma, pulmonary blastoma, renal cell carcinoma, serous carcinoma, small cell carcinoma, soft tissue carcinoma, somatostatin-secreting tumor, squamous carcinoma, squamous cell carcinoma, undifferentiated carcinoma, uveal melanoma, verrucous carcinoma, VIPoma, Wilm's tumor. In a preferred embodiment, the cancer is leukemia.

In another preferred embodiment, the cancer is a solid cancer.

"Solid cancer", as used herein, relates to an abnormal mass of tissue that usually does not contain cysts or liquid areas including carcinomas, lymphomas, and sarcoma.

In another preferred embodiment, the cancer is selected from the group consisting of colon cancer, cervical cancer, endometrial cancer, breast cancer, lung cancer and liver cancer.

"Subject", as used herein, relates to any animal (e.g., a mammal), including, but not limited to, humans, and non-human animals (including, but not limited to, non-human primates, dogs, cats, rodents, horses, cows, pigs, mice, rats, hamsters, rabbits, and the like (e.g., which is to be the recipient of a particular treatment, or from whom cells are harvested)).

In a preferred embodiment the subject is a human.

The recombinant parasporin 2 according to the invention, the nucleic acid according to the invention, the expression cassette according to the invention, the vector according to the invention, the cell according to the invention, or the pharmaceutical composition according to the invention can be administered by any suitable route of administration, for example, parenteral (e.g., intramuscular, intravenous, subcutaneous, nasal, etc.), enteral (i.e., oral, rectal, etc.), topical, etc. In a particular embodiment, the compositions for the uses of the invention are administered via an intratumoral, intraperitoneal, intrathecal, intravesical, intrapleural, intravenous, intramuscular, subcutaneous, nasal or topical route.

In a preferred embodiment, the recombinant parasporin 2 according to the invention, the nucleic acid according to the invention, the expression cassette according to the invention, the vector according to the invention, the cell according to the invention or the pharmaceutical composition according to the invention is administered intratumorally.

All the definitions and particular embodiments previously defined are equally applicable to these aspects of the invention.

The invention also relates to the following aspects:
1- A recombinant parasporin 2 protein comprising one heterologous protease cleavage site to allow the removal of the N-terminal sequence that inhibits the pore-forming ability and, optionally, a further heterologous protease cleavage site to allow the removal of the C-terminal sequence that inhibits the pore-forming ability.
2- The recombinant parasporin 2 protein according to aspect 1, further comprising a signal peptide.
3- The recombinant parasporin 2 protein according to any one of aspects 1 or 2, wherein at least one of the protease cleavage sites is the cleavage site of a protease selected from the group consisting of legumain, uPA, matriptase, furin, trypsin and human rhinovirus 3C protease.
4- The recombinant parasporin 2 protein according to any one of aspects 1 to 3, having the sequence shown in SEQ ID NO: 1, SEQ ID NO: 2, SEQ ID NO: 14, SEQ ID NO: 20 or a functionally equivalent variant thereof further comprising one heterologous protease cleavage site to allow the removal of the N-terminal sequence that inhibits the pore-forming ability and, optionally, a further heterologous protease cleavage site to allow the removal of the C-terminal sequence that inhibits the pore-forming ability.
5- The recombinant parasporin 2 protein according to aspect 4, wherein the heterologous protease cleavage site is inserted in a location after the N-terminal sequence that inhibits the pore-forming ability and/or before the C-terminal sequence that inhibits the pore-forming ability.
6- The recombinant parasporin 2 protein according to aspect 5, wherein the heterologous protease cleavage site is inserted after one of the residues selected from the group consisting of T51, Q302 and/or Q303 of SEQ ID NO: 1 or SEQ ID NO: 20, or wherein the residues from D300 to E304, from T50 to D52, and/or from T51 to D52 of SEQ ID NO: 1 or SEQ ID NO: 20 have been replaced by a protease cleavage site.
7- The recombinant parasporin 2 protein according to aspect 6, comprising a protease cleavage site after T51 and after Q302 of SEQ ID NO: 1 or SEQ ID NO: 20.
8- The recombinant parasporin 2 protein according to aspect 7, wherein the protease cleavage site is the sequence shown in SEQ ID NO: 3.
9- The recombinant parasporin 2 protein according to aspect 8 having the sequence shown in SEQ ID NO: 4.
10- The recombinant parasporin 2 protein according to aspect 7, wherein the protease cleavage site is the sequence shown in SEQ ID NO: 5.
11- The recombinant parasporin 2 protein according to aspect 10 having the sequence shown in SEQ ID NO: 6.
12- The recombinant parasporin 2 protein according to aspect 6, wherein the residues from T50 to D52 and from D300 to E304 in SEQ ID NO: 1 or SEQ ID NO: 20 have been replaced by a protease cleavage site.
13- The recombinant parasporin 2 protein according to aspect 12, wherein the protease cleavage site replacing the residues from T50 to D52 is the sequence shown in SEQ ID NO: 7 and the protease cleavage site replacing the residues from D300 to E304 is the sequence shown in SEQ ID NO: 8.
14- The recombinant parasporin 2 protein according to aspect 13 having the sequence shown in SEQ ID NO: 9.
15- The recombinant parasporin 2 protein according to aspect 6 wherein the residues from T51 to D52 have been replaced by the protease cleavage site sequence shown in SEQ ID NO: 10.
16- The recombinant parasporin 2 protein according to aspect 15 having the sequence shown in SEQ ID NO: 11.
17- The recombinant parasporin 2 protein according to aspect 6, wherein the residue T51 has been replaced by the cleavage site sequence shown in SEQ ID NO: 10 and further comprises a cleavage site sequence shown in SEQ ID NO: 12 after residue Q303.
18- The recombinant parasporin 2 protein according to aspect 17 having the sequence shown in SEQ ID NO: 13.
19- The recombinant parasporin 2 protein according to aspect 4 having the sequence shown in SEQ ID NO: 14 comprising a protease cleavage site after T27 and optionally a further protease cleavage site after Q279.
20- The recombinant parasporin 2 protein according to aspect 19, wherein the protease cleavage site after T27 has the sequence shown in SEQ ID NO: 16 and the protease cleavage site after Q279 has the sequence shown in SEQ ID NO. 12.
21- The recombinant parasporin 2 protein according to aspect 20 having the sequence shown in SEQ ID NO: 15.
22- A nucleic acid encoding a recombinant parasporin 2 protein according to any one of aspects 1 to 21.
23- An expression cassette comprising the nucleic acid according to aspect 22.
24- A vector comprising the expression cassette according to aspect 23.
25- A vector according to aspect 24 wherein the vector is a viral vector, particularly an oncolytic viral vector, more particularly an adenoviral vector.
26- A cell comprising a vector according to any one of aspects 24 or 25.
27- A pharmaceutical composition comprising a recombinant parasporin 2 protein according to any one of aspects 1 to 21, a nucleic acid according to aspect 22, an expression cassette according to aspect 23, a vector according to any one of aspects 24 to 25 or a cell according to aspect 26 and a pharmaceutically acceptable carrier or excipient.
28- A recombinant parasporin 2 according to any one of aspects 1 to 21, a nucleic acid according to aspect 22, an expression cassette according to aspect 23, a vector according to any one of aspects 24 to 25, a cell according to aspect 26 or a pharmaceutical composition according to aspect 27 for use in medicine.
29- A recombinant parasporin 2 according to any one of aspects 1 to 21, a nucleic acid according to aspect 22, an expression cassette according to aspect 23, a vector according to any one of aspects 24 to 25, a cell according to aspect 26 or a pharmaceutical composition according to aspect 27 for use in the prevention and/or treatment of cancer.
30- The recombinant parasporin 2 according to any one of aspects 1 to 21, the nucleic acid according to aspect 22, the expression cassette according to aspect 23, the vector according to any one of aspects 24 to 25, the cell according to aspect 26 or the pharmaceutical composition according to aspect 27 for use according to aspect 29, wherein the cancer is a solid cancer.
31- The recombinant parasporin 2 according to any one of aspects 1 to 21, the nucleic acid according to aspect 22, the expression cassette according to aspect 23, the vector according to any one of aspects 24 to 25, the cell according to aspect 26 or the pharmaceutical composition according to aspect 27 for use according to aspect 30, wherein the cancer is selected from the group consisting of colon cancer, cervical cancer, endometrial cancer, breast cancer, lung cancer and liver cancer.
32- The recombinant parasporin 2 according to any one of aspects 1 to 21, the nucleic acid according to aspect 22, the expression cassette according to aspect 23, the vector according to any one of aspects 24 to 25, the cell according to aspect 26 or the pharmaceutical composition according to aspect 27 for use according to any one of aspects 29 to 31, wherein the pharmaceutical composition is administered intratumorally.

### Examples

### MATERIALS AND METHODS

### Example 1: Engineering of parasporin 2 to be produced and secreted in eukaryotic cells

Figure 1 shows the structure of PS2 monomer before activation (protoxin) with the two inhibitory terminal regions in white. The loops preceding the first beta sheet (b1) and after the last beta sheet (b14) that are modified in the present invention are indicated.

The following sequence (SEQ ID NO: 1) shows underlined the inhibitory domains of PS2Aa1 that are cleaved out to activate the protoxin into a toxin. The preferred location of the protease cleavage site of the present invention is indicated between brackets. The b1 beta sheet with the sequence IREY is located after the upstream bracket and b14 beta sheets with the sequence RS is located before the downstream bracket.

MYNDKRMCSD PYQGMNKPHY CNCHTYNNSE ITGGLQVDLD 40

NQVVETFQST TDVIREYLMF NELSALSSSP ESVRSRFSSI 80

YGTNPDGIAL NNETYFNAVK PPITAQYGYY CYKNVGTVQY 120

VNRPTDINPN VILAQDTLTN NTNEPFTTTI TITGSFTNTS 160

TVTSSTTTGF KFTSKLSIKK VFEIGGEVSF STTIGTSETT 200

TETITVSKSV TVTVPAQSRR TIQLTAKIAK ESADFSAPIT 240

VDGYFGANFP KRVGPGGHYF WFNPARDVLN TTSGTLRGTV 280

TNVSSFDFQT IVQPARSLLD EQQETLEYAI PGDPSGEQLQ 320

QMEQRMFFSK CQCPKWGN* 338

A sequence comprising the open reading frame (ORF) of PS2Aa1 was purchased from Twist Bioscience. The sequence was ordered to be cloned between the CMV promoter and the SV40 polyadenylation signal (pA) of pTwist-CMV plasmid. In the sequence design the initial Methionine AA residue was substituted by a signal peptide PS2Aa1 to generate a sequence encoding a secreted protein having the sequence shown in SEQ ID NO: 2. The modified PS2Aa1 gene cloned in an expression plasmid under the control of the CMV promoter is represented in Figure 2.

The pTwistCMV-PS2Aa1 (pCMV-SP-proPS2 in Figure 2) plasmid was transfected into HEK293 human kidney cells using the calcium phosphate HEPES buffered saline (HBS) method. One day after transfection, the cell culture medium DMEM with 10% fetal bovine serum (FBS) was replaced with new medium. Two days later this medium was harvested. The harvested medium (supernatant) was incubated at 37°C during 1h with purified proteinase K (protK; 1 unit / ml) at a final concentration of 100 µg/ml to cleave the secreted parasporin 2 protoxin. ProtK was inactivated by adding 1 mM of phenylmethylsulfonyl fluoride (PMSF). The protK-treated supernatant was added to CT26 cells and pictures of cells were taken 24h later. The activation of the protoxin into toxin was observed by the formation of membrane blebs, cell swelling, and lysis (Figure 3).

Figure 3 shows CT26 colon cancer cells incubated with the protK-treated supernatant from HEK293 cells transfected with the expression plasmid of PS2Aa1. Cellular cytotoxicity induced by the toxin is observed as a cell swelling or ballooning morphological change in the cells as previously described: cells become rounded and swollen (upper image) compared to the bottom image showing untreated CT26 cells.

### Example 2: Engineering of parasporin 2 with Human Rhinovirus 3C cleavage sites

The Human Rhinovirus protease is a highly selective protease commonly used in molecular biology to eliminate peptidic TAGs from recombinant proteins, which is used in the present invention as proteolytic site to prove the concept of protease-dependent activation. Using gene fragment synthesis (Twist Biosciences) the Human Rhinovirus 3C protease cleavage LEVLFQGM (SEQ ID NO:3) site was inserted after residues T51 and Q302 of PS2Aa1 to generate a sequence encoding a secreted protein with the sequence shown in SEQ ID NO:4, shown below.

MGWSCIILFLVATATGVHSYNDKRMCSDPYQGMNKPHYCNCHTYNNSE
ITGGLQVDLDNQVVETFQSTTLEVLFQGMDVIREYLMFNELSALSSSP
ESVRSRFSSIYGTNPDGIALNNETYFNAVKPPITAQYGYYCYKNVGTV
QYVNRPTDINPNVILAQDTLTNNTNEPFTTTITITGSFTNTSTVTSST
TTGFKFTSKLSIKKVFEIGGEVSFSTTIGTSETTTETITVSKSVTVTV
PAQSRRTIQLTAKIAKESADFSAPITVDGYFGANFPKRVGPGGHYFWF
NPARDVLNTTSGTLRGTVTNVSSFDFQTIVQPARSLLDEQLEVLFQGM
QETLEYAIPGDPSGEQLQQMEQRMFFSKCQCPKWGN*

This modified PS2Aa1 was named HPS2Aa1. The modified HPS2Aa1 gene was cloned in an expression plasmid under the CMV promoter. The plasmid was transfected into HEK293 human kidney cells using the calcium phosphate HBS method. One day after transfection the cell culture medium DMEM with 10% FBS was replaced with new medium. Two days later this medium was harvested. The harvested medium (supernatant) was incubated at 37°C during 1h with purified protease HRV 3C (1 unit / ml) to cleave the modified parasporin 2 protoxin. CT26 colon cancer cells previously seeded in 96 well plates were incubated in the presence of this supernatant containing activated HPS2Aa1. After 24h the activation of the protoxin into toxin was observed by the cytotoxic effect of the HRV 3C-treated supernatant on CT26 cells (Figure 4). Cellular cytotoxicity induced by the toxin is observed as cell swelling or ballooning and membrane blebbing (upper image) compared to the bottom image showing untreated cells.

### Example 3: Engineering of parasporin 2 with a cleavage site for multiple tumor-selective proteases

Using gene fragment synthesis (Twist Biosciences), a protease cleavage site flanked by GGS flexible linkers having the sequence shown in SEQ ID NO: 5 was inserted after residues T51 and Q302 of PS2Aa1 to generate a sequence encoding a secreted protein with a sequence shown in SEQ ID NO: 6, shown below.

MGWSCIILFLVATATGVHSYNDKRMCSDPYQGMNKPHYCNCHTYNNSEITGGLQVDL
DNQVVETFQSTTGGSLSGRSDNHGGSDVIREYLMFNELSALSSSPESVRSRFSSIYG
TNPDGIALNNETYFNAVKPPITAQYGYYCYKNVGTVQYVNRPTDINPNVILAQDTLT
NNTNEPFTTTITITGSFTNTSTVTSSTTTGFKFTSKLSIKKVFEIGGEVSFSTTIGT
SETTTETITVSKSVTVTVPAQSRRTIQLTAKIAKESADFSAPITVDGYFGANFPKRV
GPGGHYFWFNPARDVLNTTSGTLRGTVTNVSSFDFQTIVQPARSLLDEQGGSLSGRS
DNHGGSQETLEYAIPGDPSGEQLQQMEQRMFFSKCQCPKWGN*

This cleavage site can be cleaved by three proteases known to be present in tumors: legumain, uPA and Matriptase. This modified PS2Aa1 was named PS2Aa1-3. The modified PS2Aa1-3 gene was cloned in an expression plasmid under the CMV promoter. The plasmid was transfected into HEK293 human kidney cells using the calcium phosphate HBS method. One day after transfection the cell culture medium DMEM with 10% FBS was replaced with new medium. Two days later this medium was harvested. CT26 colon cancer cells and HepG2 hepatocarcinoma cells, known to express uPA and legumain, were previously seeded in 96 well plates and were incubated in the presence of the harvested medium (supernatant) with PS2Aa1-3. After 24h the activation of the protoxin PS2Aa1-3 into toxin was observed by the cytotoxic effect of the supernatant on each of the cell lines (see Figure 5 for CT26 cells and Figure 6 for HepG2 cells). Cellular cytotoxicity induced by the toxin is observed as cell swelling or ballooning and membrane blebbing (upper image) compared to the bottom image showing untreated cells.

In another human tumor cell line, the cervical carcinoma cell line HeLa, the effect of the harvested medium (supernatant) containing PS2Aa1-3 was not evident, presumably due to lower levels of proteases in HeLa cells. Incubation of the supernatant with purified matriptase resulted in the treated supernatant having a strong cytotoxicity against HeLa cells, indicating that the modified PS2Aa1-3 can be activated by matriptase (Figure 7). Cellular cytotoxicity induced by the toxin is observed as cell swelling or ballooning and membrane blebbing (upper image) compared to the bottom image showing untreated cells.

### Example 4: Engineering of parasporin 2 with a cleavage site for trypsin

Using gene fragment synthesis (Twist Biosciences), residues from D300 to E304 of PS2Aa1 were replaced by the protease cleavage site having the sequence SGRS (SEQ ID NO: 7) , and the residues from T50 to D52 were replaced by a protease cleavage site having the sequence SGRSG (SEQ ID NO: 8) to generate a sequence encoding a secreted protein with the sequence shown in SEQ ID NO: 9 shown below.

MGWSCIILFLVATATGVHSYNDKRMCSDPYQGMNKPHYCNCHTYNNSEITGGLQV
DLDNQVVETFQSSGRSVIREYLMFNELSALSSSPESVRSRFSSIYGTNPDGIALNNET
YFNAVKPPITAQYGYYCYKNVGTVQYVNRPTDINPNVILAQDTLTNNTNEPFTTTITI
TGSFTNTSTVTSSTTTGFKFTSKLSIKKVFEIGGEVSFSTTIGTSETTTETITVSKSVTVT
VPAQSRRTIQLTAKIAKESADFSAPITVDGYFGANFPKRVGPGGHYFWFNPARDVL
NTTSGTLRGTVTNVSSFDFQTIVQPARSLLSGRSGTLEYAIPGDPSGEQLQQMEQR
MFFSKCQCPKWGN*

This modified PS2Aa1 was named PS2Aa1-1. The modified PS2Aa1-1 gene was cloned in an expression plasmid under the control of the CMV promoter. The plasmid was transfected into HEK293 human kidney cells using the calcium phosphate HBS method. One day after transfection the cell culture medium DMEM with 10% FBS was replaced with new medium. Two days later this medium was harvested. CT26 colon cancer cells were previously seeded in 96 well plates and were incubated in the presence of the harvested medium (supernatant) containing PS2Aa1-1. Incubation of the supernatant with purified trypsin revealed a strong cytotoxicity, indicating that the modified PS2Aa1-1 can be activated by trypsin (Figure 8). Cellular cytotoxicity induced by the toxin is observed as cell swelling or ballooning and membrane blebbing (upper image) compared to the bottom image showing untreated cells.

### Example 5: Engineering of parasporin 2 with cleavage sites for furin

Using gene fragment synthesis (Twist Biosciences) the residue T51 of PS2Aa1 was replaced by the protease cleavage site having the sequence QRLRR (SEQ ID NO: 10) to generate a sequence encoding a secreted protein with the sequence shown in SEQ ID NO: 11 shown below.

MGWSCIILFLVATATGVHSYNDKRMCSDPYQGMNKPHYCNC
HTYNNSEITGGLQVDLDNQVVETFQSTQRLRRDVIREYLMFN
ELSALSSSPESVRSRFSSIYGTNPDGIALNNETYFNAVKPPITAQ
YGYYCYKNVGTVQYVNRPTDINPNVILAQDTLTNNTNEPFTTT
ITITGSFTNTSTVTSSTTTGFKFTSKLSIKKVFEIGGEVSFSTTIGT
SETTTETITVSKSVTVTVPAQSRRTIQLTAKIAKESADFSAPITVD
GYFGANFPKRVGPGGHYFWFNPARDVLNTTSGTLRGTVTNV
SSFDFQTIVQPARSLLDEQQETLEYAIPGDPSGEQLQQMEQR
MFFSKCQCPKWGN*

This modified PS2 was named PS2Aa1-Fu.

In a different design, using gene fragment synthesis (Twist Biosciences), the residue T51 of PS2Aa1 was replaced by the protease cleavage site having the sequence QRLRR (SEQ ID NO: 10) and the protease cleavage site having the sequence RRKR (SEQ ID NO: 12) was inserted after Q303 of parasporin 2 to generate a sequence encoding a secreted protein having the sequence shown in SEQ ID NO: 13, shown below.

MGWSCIILFLVATATGVHSYNDKRMCSDPYQGMNKPHYCNC
HTYNNSEITGGLQVDLDNQVVETFQSTQRLRRDVIREYLMFN
ELSALSSSPESVRSRFSSIYGTNPDGIALNNETYFNAVKPPITAQ
YGYYCYKNVGTVQYVNRPTDINPNVILAQDTLTNNTNEPFTTT
ITITGSFTNTSTVTSSTTTGFKFTSKLSIKKVFEIGGEVSFSTTIGT
SETTTETITVSKSVTVTVPAQSRRTIQLTAKIAKESADFSAPITVD
GYFGANFPKRVGPGGHYFWFNPARDVLNTTSGTLRGTVTNV
SSFDFQTIVQPARSLLDEQQRRKRETLEYAIPGDPSGEQLQQM
EQRMFFSKCQCPKWGN*

This cleavage site can be cleaved by furin present in the Golgi of eukaryotic cells. Therefore, without wishing to be bound by theory, the secreted PS2Aa1 is expected to be secreted in an already cleaved or active form. This modified PS2Aa1 was named PS2Aa1-Fu2. The modified PS2Aa1-Fu and PS2Aa1-Fu2 genes were cloned in expression plasmids under the CMV promoter. The plasmids were transfected into HEK293 human kidney cells using the calcium phosphate HBS method. One day after transfection, the cell culture medium DMEM with 10% FBS was replaced with new medium. Two days later this medium was harvested. CT26 colon cancer cells were previously seeded in 96 well plates and were incubated in the presence of this medium containing PS2Aa1-Fu or PS2Aa1-Fu2. After 24h, the activation of the protoxin PS2Aa1-Fu or PS2Aa1-Fu2 into toxin was observed by the cytotoxic effect of the treated supernatant (Figure 9). Cellular cytotoxicity induced by the toxin is observed as cell swelling or ballooning and membrane blebbing (upper image) compared to the bottom image showing untreated cells.

The cytotoxic activity of PS2Aa1-Fu2, activated by furin during the secretory pathway, was compared to the activity of PS2Aa1-3, activated by legumain, uPA or matriptase proteases in the extracellular medium. For this experiment, HEK293 cells were transfected in parallel with the expression plasmids of the respective modified parasporins using the same conditions. Supernatants were obtained in parallel and added to CT26 cells at two different dilutions in complete medium (DMEM, 10% FBS). After a 16h incubation, the cytotoxicity was evaluated using a microscope. Upper panels: No clear cytotoxicity was observed from the diluted supernatants containing PS2Aa1-3. Bottom: CT26 colon cancer cells incubated with the supernatant from HEK293 cells transfected with the expression plasmid of PS2Aa1-Fu2 at 4x and 16x dilutions. Cytotoxicity is observed with the characteristic morphological changes associated to parasporin-2 cytotoxicity as described for figure 3 (Figure 10).

A different type of experimental proof of the antitumor cytotoxic activity of the modified parasporins PS2Aa1-Fu2, was obtained by mixing cells producing the protoxin with unmodified bystander target tumor cells. For this experiment, the pCMV-PS2Aa1-Fu2 plasmid was transfected into HEK293-GL human kidney cells using the calcium phosphate HBS method. Control HEK293-GL transfected cells were prepared by transfection using the pCMV-PS2Aa1 plasmid, which encodes the secreted wild type PS2Aa1 that needs proteinase K to be activated. HEK293-GL is a derivative of HEK293 that expresses the green fluorescent protein and luciferase. One day after transfection, the cell culture medium DMEM with 10% FBS was replaced with new medium. One day later, the PS2Aa1-Fu2-transfected HEK293-GL cells were trypsinized and seeded in new wells together with a 10-fold excess of CT26 target cells as a co-culture. The co-culture was incubated for two days and pictures were taken under fluorescence and bright field at the microscope. The coculture of PS2Aa1-Fu2 transfected cells with CT26 target cells showed the complete lysis of target cells (Figure 11 upper image) compared to a coculture of PS2Aa1-transfected cells with CT26 (Figure 11 lower image).

### Example 6- Engineering of PS2Ab with cleavage sites for furin

A sequence comprising the ORF of PS2Ab with furin sites inserted to remove the N-terminal and C-terminal inhibitory domains and with the initial methionine substituted by a signal peptide was purchased from Twist Bioscience. The furin sequence RLRR (SEQ ID NO: 16) was inserted after T27, and the sequence RRKR (SEQ ID NO: 12) was inserted after Q279, all referring to SEQ ID NO: 20. The sequence was cloned between the CMV promoter and the SV40 polyadenylation signal (pA) of pTwist-CMV plasmid. The final ORF encodes a secreted protein having the sequence shown in SEQ ID NO: 15.

### PS2AbFu2 (SEQ ID NO: 15)

MGWSCIILFLVATATGVHSYYTTQVTGGFQADLNNQVVETFQPST
RLRRNVIQEYLTFNDLPALGSSPQSVRSRFSSIYGTNPDGIALNN
ETYFSAVQPPITVQYGHYCYKNVGTVQYVNRPTDINPNVILAQDT
LTNNTNEPFTTTITLTGSWTKSSTVTSSTTTGLKITTKLSIKKVF
EIGGEVSFSTTIGSSEATSETFTVSKAVTVTVPAQSRRNIQLTAK
IARESADFSAPITVDGYFGANFPRRVGPGGHYFWFNPARDVLNAT
SGTLRGTVTNVSSFDFQTVVQPAYSLLAEQQRRKREALESAISGD
PSEEQLKQIQQTIGL*

The pTwistCMV-PS2AbFu2 plasmid was transfected into HEK293 human kidney cells using the calcium phosphate HBS method. One day after transfection the cell culture medium DMEM with 10% FBS was replaced with new medium. Two days later this medium was harvested. CT26 colon cancer cells were previously seeded in 96 well plates and were incubated in the presence of the harvested medium (supernatant) containing PS2AbFu2. After 24h the activation of the protoxin PS2AbFu2 into toxin was observed by the cytotoxic effect of the supernatant on CT26 cells (Figure 12). Cellular cytotoxicity induced by the toxin is observed as cell swelling or ballooning and membrane blebbing (upper image) compared to the bottom image showing untreated cells.

### Example 7: In vivo antitumor activity of a modified parasporin 2.

The pCMV-PS2Fu2 plasmid is transfected into HEK293 human kidney cells using the calcium phosphate HBS method. One day after transfection the cell culture medium DMEM with 10% FBS is replaced with new medium. One day later this complete medium is replaced with serum-free medium (Optimem, Gibco). Two days later this conditioned medium or supernatant containing the secreted PS2AbFu2 toxin is harvested. In parallel a supernatant control was produced from HEK293 cells transfected with a control plasmid. CMT64 mouse lung cancer cells were injected in C57BL/6 immune-competent mice subcutaneously and allowed to form tumors. When tumors reached a volume of 0.1cc were injected with 20 microliters of the supernatant containing the toxin or the supernatant control. Each treatment group, PS2AbFu2 or control, contained 5 mice with tumors. Tumor volume was followed during time. Figure 13 shows that tumors injected with the supernatant containing the modified parasporin-2 toxin PS2Fu2 were inhibited in their growth compared to the ones treated with the control.

### Example 8: Antitumor activity of an oncolytic adenovirus encoding a modified parasporin 2.

PS2Ab-Fu2 expression cassette composed of an splicing acceptor (SA) and the DNA sequence encoding a modified parasporin-2 was cloned after the fiber gene of the oncolytic adenovirus ICOVIR15K using standard techniques Rojas J.J. et al., Gene Ther . 2012 Apr;19(4):453-7. The scheme of the oncolytic adenovirus ICOVIR-15K-SA-PS2-Fu is shown in figure 14. This virus produces a secreted modified PS2-Ab toxin that is activated upon cleavage with furin. This activation leads to the oligomerization of the protein to form pores in the membrane of eukaryotic cells. To demonstrate the antitumor activity of the toxin derived from this oncolytic virus A549 human lung tumor cells were infected with the virus and three days later the supernatant was collected. A parallel infection was performed with ICOVIR15K as a control. The harvested supernatants were added to human T cell leukemia Jurkat cells. Twenty hours later pictures were taken (figure 15). Figure 15 shows that the supernatants from A549 cells infected with ICOVIR-15K-SA-PS2Fu induced blebbing and cytoplasm release in Jurkat cells compared to the SN of ICOVIR15K. Cellular cytotoxicity induced by the toxin is observed as cell swelling or ballooning and membrane blebbing (upper image) compared to the bottom image showing untreated cells.

## Claims

1. A recombinant parasporin 2 protein comprising one heterologous protease cleavage site to allow the removal of the N-terminal sequence that inhibits the pore-forming ability and, optionally, a further heterologous protease cleavage site to allow the removal of the C-terminal sequence that inhibits the pore-forming ability.

2. The recombinant parasporin 2 protein according to claim 1, further comprising a signal peptide.

3. The recombinant parasporin 2 protein according to any one of claims 1 or 2, wherein at least one of the protease cleavage sites is the cleavage site of a protease selected from the group consisting of legumain, uPA, matriptase, furin, trypsin and human rhinovirus 3C protease.

4. The recombinant parasporin 2 protein according to any one of claims 1 to 3, having the sequence shown in SEQ ID NO: 1, SEQ ID NO: 2, SEQ ID NO: 14, SEQ ID NO: 20 or a functionally equivalent variant thereof further comprising one heterologous protease cleavage site to allow the removal of the N-terminal sequence that inhibits the pore-forming ability and, optionally, a further heterologous protease cleavage site to allow the removal of the C-terminal sequence that inhibits the pore-forming ability.

5. The recombinant parasporin 2 protein according to claim 4, wherein the heterologous protease cleavage site is inserted in a location after the N-terminal sequence that inhibits the pore-forming ability and/or before the C-terminal sequence that inhibits the pore-forming ability.

6. The recombinant parasporin 2 protein according to claim 5, wherein the heterologous protease cleavage site is inserted after one of the residues selected from the group consisting of T51, Q302 and/or Q303 of SEQ ID NO: 1 or SEQ ID NO: 20, or wherein the residues from D300 to E304, from T50 to D52, and/or from T51 to D52 of SEQ ID NO: 1 or SEQ ID NO: 20 have been replaced by a protease cleavage site.

7. The recombinant parasporin 2 protein according to claim 6, comprising a protease cleavage site after T51 and after Q302 of SEQ ID NO: 1 or SEQ ID NO: 20.
preferably wherein the protease cleavage site is the sequence shown in SEQ ID NO: 3, more preferably wherein the recombinant parasporin 2 has the sequence shown in SEQ ID NO: 4, or
preferably wherein the protease cleavage site is the sequence shown in SEQ ID NO: 5, more preferably wherein the recombinant parasporin 2 has the sequence shown in SEQ ID NO: 6.

8. The recombinant parasporin 2 protein according to claim 6, wherein the residues from T50 to D52 and from D300 to E304 in SEQ ID NO: 1 or SEQ ID NO: 20 have been replaced by a protease cleavage site, preferably the protease cleavage site replacing the residues from T50 to D52 is the sequence shown in SEQ ID NO: 7 and the protease cleavage site replacing the residues from D300 to E304 is the sequence shown in SEQ ID NO: 8, more preferably, the recombinant parasporin 2 has the sequence shown in SEQ ID NO: 9.

9. The recombinant parasporin 2 protein according to claim 6, wherein the residues from T51 to D52 have been replaced by the protease cleavage site sequence shown in SEQ ID NO: 10, preferably the recombinant parasporin 2 has the sequence shown in SEQ ID NO: 11.

10. The recombinant parasporin 2 protein according to claim 6, wherein the residue T51 has been replaced by the cleavage site sequence shown in SEQ ID NO: 10 and further comprises a cleavage site sequence shown in SEQ ID NO: 12 after residue Q303, preferably the recombinant parasporin 2 protein has the sequence shown in SEQ ID NO: 13.

11. The recombinant parasporin 2 protein according to claim 5, having the sequence shown in SEQ ID NO: 14 comprising a protease cleavage site after T27 and optionally a further protease cleavage site after Q279, preferably the protease cleavage site after T27 has the sequence shown in SEQ ID NO: 16 and the protease cleavage site after Q279 has the sequence shown in SEQ ID NO. 12, more preferably the recombinant parasporin 2 has the sequence shown in SEQ ID NO: 15.

12. A nucleic acid encoding a recombinant parasporin 2 protein according to any one of claims 1 to 11, an expression cassette comprising the nucleic acid, a vector comprising the expression cassette or a cell comprising the vector, preferably the vector is a viral vector, particularly an oncolytic viral vector, more particularly an adenoviral vector.

13. A pharmaceutical composition comprising a recombinant parasporin 2 protein according to any one of claims 1 to 11, a nucleic acid, an expression cassette, a vector or a cell according to claim 12 and a pharmaceutically acceptable carrier or excipient.

14. A recombinant parasporin 2 according to any one of claims 1 to 11, a nucleic acid, an expression cassette, a vector, a cell according to claim 12 or a pharmaceutical composition according to claim 13 for use in medicine.

15. A recombinant parasporin 2 according to any one of claims 1 to 11, a nucleic acid, an expression cassette, a vector, a cell according to claim 12 or a pharmaceutical composition according to claim 13 for use in the prevention and/or treatment of cancer, preferably the cancer is a solid cancer, preferably the cancer is selected from the group consisting of colon cancer, cervical cancer, endometrial cancer, breast cancer, lung cancer and liver cancer.
